**Europäisches Patentamt**

**European Patent Office**

**Office européen des brevets**

(19)

(11) **EP 1 228 769 A1**

(12) **EUROPÄISCHE PATENTANMELDUNG**

(43) Veröffentlichungstag:
07.08.2002 Patentblatt 2002/32

(51) Int Cl.⁷: **A61K 45/06**, A61P 43/00

(21) Anmeldenummer: 01102384.3

(22) Anmeldetag: 02.02.2001

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(71) Anmelder: **Schür, Jörg-Peter, Prof.
41844 Wegberg-Dalheim (DE)**

(72) Erfinder: **Schür, Jörg-Peter, Prof.
41844 Wegberg-Dalheim (DE)**

(74) Vertreter: **Helbing, Jörg, Dr. Dipl.-Chem. et al
Patentanwälte
von Kreisler-Selting-Werner,
Postfach 10 22 41
50462 Köln (DE)**

(54) **Symbiontisches Regenerativum**

(57)     Die vorliegende Erfindung betrifft ein regeneratives Arzneimittel zur natürlichen infektionsimmunologischen Therapie mit Induktions-, Regulations- und Effektormechanismen zur frühen, natürlichen, zellulären Immunabwehr bei Mensch und Tier, umfassend eine spezielle Zusammensetzung mit einer Mikroorganismuskomponente und einer Modulatorkomponente, Nahrungsmittel, Nahrungsergänzungsmittel, Tiernahrungsmittel und Tiernahrungsergänzungsmittel, enthaltend diese Zusammensetzung, und die Verwendung dieser Zusammensetzung zur Herstellung von regenerativen Mitteln für die Behandlung von Mensch und Tier.

EP 1 228 769 A1

**Beschreibung**

Die vorliegende Erfindung betrifft ein regeneratives Arzneimittel zur natürlichen infektionsimmunologischen Therapie mit Induktions-, Regulations- und Effektormechanismen zur frühen, natürlichen, zellulären Immunabwehr bei Mensch und Tier, umfassend eine spezielle Zusammensetzung mit einer Mikroorganismuskomponente und einer Modulatorkomponente, Nahrungsmittel, Nahrungsergänzungsmittel, Tiernahrungsmittel und Tiernahrungsergänzungsmittel, enthaltend diese Zusammensetzung, und die Verwendung dieser Zusammensetzung zur Herstellung von regenerativen Mitteln für die Behandlung von Mensch und Tier.

**Hintergrund der Erfindung**

Der Dickdarm ist stark besiedelt mit Mikroorganismen ($10^{10}$ bis $10^{12}$ pro g Inhalt) und ist der am stärksten besiedelte Teil des menschlichen bzw. tierischen Verdauungstraktes und des Körpers überhaupt. Im Dünndarm sind weniger Mikroorganismen (etwa $10^4$ bis $10^8$ Organismen/ml). Der pH-Wert 1 - 2 im Magen ermöglicht praktisch kein Leben von Bakterien, sodass dort nur noch $10^1$ bis $10^3$ Organismen/ml leben (Organismen im Darmtrakt, Tannock, 1995).

Das saure Milieu im Magen ist somit eine wichtige Barriere gegen Krankheitserreger und fremde Organismen. Zu diesen gehören auch die sogenannten "Probiotika". Einzelne Mikroorganismen und/oder Gemische zumeist aus der Gruppe "apathogener" Lactobazillen (Acidophilus, Bifidus u. ä.), die neuerlich als Nahrungsergänzungsmittel angeboten und verwendet werden, manchmal auch in Kombinationen mit Vitaminen und/oder Mineralstoffen/Spurenelementen oder Komplexen daraus. Es wurde festgestellt, dass sogenannte "probiotische Stämme" (apathogene Mikroorganismen z. B. Gruppe Lactobazillen) sich sehr schwer im Darm festsetzen können, d. h. eine Kolonisierungsresistenz oder Barriereneffekt besteht (Tannock, 1995).

Es ist somit fraglich, ob ein einzelner Stamm oder Gemische einer einzigen Gruppe (apathogene Lactobazillen) einen günstigen Einfluss bzw. eine Immunabwehr-Aktivierung ausüben (Tannock, 1998).

Da die natürliche Darmflora eine wichtige Rolle im Abwehrsystem von Mensch und Tier gegen unerwünschte Krankheitserreger und sonstige Mikroorganismen spielt, wird dies besonders erkennbar, wenn sie durch falsche Ernährung und/oder Gabe von Medikamenten gestört wird.

Fehlernährung, Anwendung von Antiinfektiva, Immunsuppressiva, Antidepressiva, Kontrazeptiva, Antiallergika führen dazu, dass zum einen Resistenzen von Mikroorganismen gefördert und zum anderen assoziierte Krankheiten generiert werden, deren Symptome ursächlich oft damit nicht in Verbindung gebracht werden, da sie andere Krankheitsbilder vortäuschen. So nehmen z. B. "Pilzinfektionen" z. B. der Gattungen Candida und Aspergillus in den letzten Jahren erheblich zu und die oben beschriebenen Krankheitsbilder sind mit großer Wahrscheinlichkeit ursächlich mit Candida- und/oder Aspergillus-Kontaminationen in Schleimhaut und Gastrointestinaltrakt zu sehen, zumal da Hefepilze und Pilze dieser Gattungen in der Lage sind, auch mutagene, cancerogene Toxine zu bilden, die dann wiederum zu zum Teil nicht nur lebensqualitätsmindernden sondern zu lebensbedrohenden Auswirkungen führen. Bereits 1980 beschreibt H. J. Preusser (Medical Mycology Zbl. Bact. Suppl. 8) die Bedeutung des "pathogenic potential in the genus Candida" und Hurley, R./De Louvois berichten über die "ecological aspects of yeast-like funghi of medical importance". Male, O./Boltz-Nitulescu beschreiben bereits in der Wien. Klin. Wochenschr. 91, 826 - 830 (1979), "tierexperimentelle Studien über die Persorption von Candida Albicans und die intestinale Auslösbarkeit einer Immunantwort". Büchner, Th. beschreibt in "Pilzinfektionen in der Onkologie", Schattauer, 1996, über prädisponierende Faktoren für Mykosen: hormonelle Erkrankungen, Therapie: Kortikosteroide; gastroenterologische Erkrankungen: Therapie Immunsuppressiva; hämatologische Erkrankungen, Therapie: Zytostatika; Immundefekte (u. a. AIDS), Therapie: Bestrahlung; maligne Tumore, Therapie: Antibiotika; Infektionskrankheiten, Verweilkrankheiten, Verbrennungen, Großchirurgie.

Die Empfänglichkeit für Krankheiten und/oder Infektionen wird durch die genannte Fehlernährung bzw. die Anwendung von Antiinfektiva praktisch erhöht. Somit hat die Darmflora eine der wichtigsten Schutzfunktionen im Körper:

- Verdrängung von gesundheitsschädigenden Bakterien durch Konkurrenz vor Ort (Koionisierungsresistenz/Barriereneffekt);
- Produktion von organischen Säuren (Milchsäure, Essigsäure, Buttersäure, Propionsäure) und Bacteriocinen, die mikrobizid wirken;
- Stimulation des Immunsystems durch Erhöhung der Aktivität von Killerzellen, Lymphozyten, Macrophagen (zelluläre Immunantwort) und erhöhte Produktion von Zytokinen (Sekretion) und Antikörper (humorale Immunantwort).

So wurde z. B. in Studien festgestellt, dass die Krebsaktivität (z. B. bei malignen Tumoren) abnahm, je mehr Immunreaktionen unter Erhöhung der T-Zellen-Stimulation und Zytokinsekretion auftraten (K. F. Kölmel, Melanoma Research 2, pp. 207 - 211 (1992)). Da "normale" Medikamente wie z. B. Immunsuppressiva (z. B. Kortikosteroide) die T-Lymphozyten-Bildung schwächen, die Zahl der Eosinophilen (spez. weiße Blutkörperchen) verhindert, T-Suppression-Zellen unterdrückt, ist klar, dass bei heutiger Medikation (z. B. Allergien) dieser und anderer Präparate, die das Immunsystem mehr oder weniger stark beeinflussen und/oder eine "Fremdpopulation" (z. B. Selektionismus) im Gastrointestinaltrakt durch z. B. Mykosen und deren hochgradig gefährliche Toxinbildungen (z. B. Aspergillus parasiticus - Aflatoxin - cancerogenes Toxin) ermöglichen oder gar provozieren (Ziemer und Gibson, 1998), wir inzwischen einer neuen Generation von regenerativen Arzneimitteln benötigen, die natürlich, ursächlich ihre Wirkung und Arbeit entfalten bzw. verrichten.

60 % der gesamten Antikörper des menschlichen und tierischen Organismus werden im Gastrointestinaltrakt hergestellt (Scientific Concepts of Functional Food in Europe, 1999). Die Erreger, die die Abwehrmechanismen im Nasen-, Mund-, Rachenraum und der Speiseröhre, des Magens (pH 1 - 2), Gallensäure und Enzyme der Bauchspeicheldrüse überwunden haben, können sich im gesunden Körper nirgends festsetzen, da der Darm so stark mit körpereigenen Mikroorganismen-Rasen besiedelt ist, dass für ein "Andocken" wenig Platz bleibt. Makrophagen und natürliche Killerzellen (NK) sind wesentliche Elemente der "First Line of Defense", d. h. der natürlichen, antigen-unspezifischen zellulären Infektabwehr.

Im gesunden Organismus haben diese Elemente großen Einfluss auf die Quantität der anschließenden adaptiven, antigen-spezifischen Abwehrphase. Das natürliche Immunsystem ist stets in der Lage, reaktivierende Abwehrleistungen zu erbringen, im Gegensatz zur adaptiven - erworbenen oder genetisch bedingten - Immundefizienz. Selbst in Abwesenheit funktioneller T- und B-Zellen kann das Immunsystem "post infectionem" mit z. B. fakultativen, intrazellulären Bakterien wie Listeria monocytogenes Makrophagen (zellulär), aktivierendes Interferon (y) produzieren und damit die bakterielle Replikation hemmen. Die Quelle hierfür sind die natürlichen Killerzellen, welche von erregerstimulierten Makrophagen aktiviert wurden. Dieser Mechanismus der erregerinduzierten Aktivierung der natürlichen, zellulären Abwehr konnte mit einer Vielzahl von Mikroorganismen bestätigt werden. Eine Interaktion von Makrophagen und natürlichen Killerzellen wird wahrscheinlich nicht durch Zytokinsekretion, sondern auch durch membranständige Liganden vermittelt.

Im übrigen sind Makrophagen, die es in verschiedenen Bereichen des Organismus gibt, die Fresszellen (antigenrepräsentierend bzw. -produzierend) des Abwehrsystems, jedoch können auch einige Erreger in diesen Makrophagen überleben und sich in Zellulärsystem vermehren. Das Abtöten von in Makrophagen eingeschleusten Erregern geschieht durch Bildung reaktiver Sauerstoffradikale, z. B. durch Zytokine bzw. deren Gene (Humorale = (Re)aktivierung von Makrophagen). Aktivierte Makrophagen können auch tumorizid sein.

Um das Immunsystem zu trainieren, muss ein ständiger Stoffwechseltransport aus dem zum lymphatischen Abwehrsystem gehörenden Darmschleimhautgewebe (Mukosa) in den Körper (Körperzellen) stattfinden, um das körpereigene Immunsystem zu aktivieren. Ebenso ernähren nützliche Bakterien die Mukosa mit z. B. Vitaminen etc., um die Darmtätigkeit zu regulieren. So erscheint eine regenerative Immunregulation mit symbiontischen Mikroorganismen und dazugehörigen notwendigen Stoffen sinnvoll und effektiv, zumal im menschlichen und tierischen Körper Nase, Mund, Hals, Dünndarm, Dickdarm, Genital- und Schleimhautbereiche mit lebensnotwendigen Mikroorganismen (ca. 100 Billionen) besiedelt sind. Hier leben 400 - 500 verschiedene Arten, vorherrschend im Dickdarm sind Bacteroides, Eubacterium, Bifidobacterium, Clostridium, Enterococcus, Fusobacterium, Peptostreptococcus, Ruminococcus, Lactobacillus und Escherichia coli.

Im Stand der Technik sind bereits Zusammensetzungen bekannt (als Arzneimittel oder Nahrungsergänzungsmittel), in denen ein wesentlicher Bestandteil eine vermehrungsfähige Mikroorganismuskultur ist. So enthält das Nahrungsergänzungsmittel "Merck Bion 3" 3 Lactobazillen (Acidophilus, Bifidus, Bifidolongum), 13 Vitamine und 14 Mineralstoffe und Spurenelemente (s. DE-A-19830528 und EP-A-0 931 543). B. J. Rembach et al. berichten in The Lancet, Vol. 354, 635 - 639 (1999), dass Colitis ulcerosa ("IBO Inflammation Bowel Diseases") mit E. coli-Mikroorganismen erfolgreich behandelt wurde. Weiterhin berichtet K. F. Kölmel in Melanoma Res., Vol. 9, S. 511 - 519 (1999), dass "... Lysatgabe" unter Ansteigen von Fieber, TNF-$\alpha$-Spiegel im Serum, Konz. von Thrombin/Antithrombin III Komplex/Fibrin von Streptokokken und Serratia marcescens zu erhöhter Immunität bei der Abwehr/Abnahme von malignen (Haut)-Tumoren führt (case studies).

EP-A-0904784 (Unilever Research) ist offenbart, dass fungiziden Schutz und geringeren Pilzbefall (Verderb) bei Nahrungsmitteln durch eine Behandlung der Nahrungsmitteln mit verschiedenen Lactobazillen +/- 1 oder mehreren Hefen (z. B. Saccharomyces cerevisiae, GRAS-Stämme, Pediococci, Propionib. oder Leuconosto) einzeln oder kombiniert erzielt werden kann. Auch Nahrungsmittel oder quasi Medikationen zur Verbesserung gastrointestinaler Probleme werden erwähnt.

WO 99/49875 berichtet über die Bekämpfung intestinaler Probleme oder Krankheitsbilder durch die Gabe von Antibiotika. Hierbei werden Lactobazillen, Saccharomyces (Hefen) und Enterokokken gemeinsam und in Kombination mit den Antibiotika verabreicht, um deren intestinale Seiteneffekte zu verringern bzw. zu verbessern.

Zwar wird für einige der vorstehend beschriebenen Zusammensetzungen erwähnt, dass mit der Gabe von Mikroorganismen ein gewisser pharmakologischer/gastrointestinaler Effekt verbunden ist. Dieser Effekt ist jedoch noch nicht so stark, dass eine tatsächliche Regeneration eines geschädigten menschlichen bzw. tierischen Organismus möglich ist. Beispiele sind Gaben von teilweise pathogenen Mikroorganismen wie E. coli, Streptokokken und Serratia-Erreger, die ähnlich wie Impfungen auschließlich eine Antikörper-/Antigen-Reaktion unter evtl. entstehenden Begleiterscheinungen (z. B. Fieber, Unwohlsein u. ä.) klinische Begleitung notwendig machen. Eine "Standard"-Medikation ist hiermit nicht möglich.

Die Aufgabe der vorliegenden Anmeldung bestand nun darin, ein Arzneimittel bzw. Nahrungsergänzungsmittel zur Verfügung zu stellen, das eine regenerative Wirkung auf geschädigte Kulturen von Mikroorganismen und deren Funktionen (bzw. geschädigtem Stoffwechsel) in Mensch und Tier aufweist. Überraschenderweise wurde im Rahmen der Entwicklungsarbeiten und bakteriologischen Tests zu der parallelen Anmeldung EP 00124497 beobachtet, dass gewisse Stoffe, wie z.B. GRAS-Aromastoffe, nicht nur protagonistisch dekontaminative, mikrobizide Eigenschaften aufweisen, sondern auch in der Lage sind, genau das Gegenteil davon zu tun, nämlich antagonistisch regenerative Eigenschaften zu entfalten, d. h., Mikroorganismenwachstum zu stabilisieren bzw. zu erhalten oder gar zu fördern. Darauf aufbauend wurde nun gefunden, dass durch eine Verabreichung einer Kombination aus einem speziellen Mikroorganismuskomponente und einer speziellen Modulatorkomponente geschädigte Kulturen von Mikroorganismen und deren Funktionen (bzw. geschädigtem Stoffwechsel) regeneriert werden können.

Die Wirkung des erfindungsgemäßen Arzneimittels (nachfolgend auch " symbiontisches Regenerativum" genannt) beruht darauf, dass es Synergismen von Substanzgruppen in Verbindung mit Mikroorganismen (möglichst aller Art) existieren, die eine wachstumsfördernde, stimulierende oder kolonisierende Eigenschaft besitzen, sodass diese Mikroorganismen dort "andocken", wo sie gegebenenfalls eingesetzt und/oder benötigt werden und dabei nicht während der Einwirkung absterben, sondern vermehrt ihre Eigenschaften dort entwickeln, d. h., permeieren oder penetrieren können. Es ist dabei einerseits möglich, gutartige Mikroorganismen wie z. b. Probiotika (Lactobazillen) dort kolonisieren zu lassen, sodass sie in der Lage sind, wieder ein gesundes Milieu herzustellen. Andererseits können dadurch fakultativ pathogene und/oder pathogene Mikroorganismen in geringeren Mengen (mit/ohne gutartige(n) Mikroorganismen) effektiv an den Ort der therapeutischen Notwendigkeit "angedockt" werden, um seine Wirkung zu entfalten. Dies kann dann auch eine schwach ausgeprägte Antikörper-/-Antigen-Wirkung sein.

Es hat sich herausgestellt, dass sowohl Aromastoffe bzw. deren synergistische Gemische als auch Enzyme bzw. deren synergistische Gemische oder Pflanzenstoffe bzw. deren synergistische Gemische solche Kolonisierungen, Stimulierungen bzw. Andock-Funktionen durch symbiontische Synergismen steigern, d. h. in Verbindung mit den Mikroorganismen regenerative Eigenschaften entwickeln.

## Zusammenfassung der Erfindung

Gegenstand der vorliegenden Anmeldung ist dem gemäß

(1) Regeneratives Arzneimittel umfassend

(a) eine Mikroorganismuskomponente, enthaltend:

(a1) wenigstens einen Typ apathogener Mikroorganismen und/oder
(a2) wenigstens einen Typ pathogener oder fakultativ pathogener Mikroorganismen;

(b) eine Modulatorkomponente, enthaltend

(b1) wenigstens ein Enzym;
(b2) wenigstens einen GRAS(Generally Recognized As Safe)-Aromastoff oder ein Derivat davon und/oder
(b3) wenigstens einem tierischen oder pflanzlichen Extrakt oder ein Derivat davon, der/das eine Verbindung mit alkoholischer, Säure-, Ester, Aldehyd-, Acetal- , phenolischer, funktionellen Gruppen und/oder mit Doppelbindungen umfasst;

(2) eine bevorzugte Ausführungsform des Arzneimittels (1), wobei das Arzneimittel inhalativ, oral, intravenös, intramuskulär, rektal, Kontaktpräparat, innerlich/äußerlich (auch Schleimhaut), intraperitoneal, subkutan, auf/in inneren Organen (z. B. endoskopisch) in Form von Tabletten, flüssig, Gas, Pulver, Injektion, Infusion, Suppositorium, Spray, Salben oder Pflaster appliziert werden kann;
(3) die Verwendung der in (1) definierten Zusammensetzung aus Mikroorganismuskomponente (a) und Modulatorkomponente (b) zur Herstellung eines dekontaminativen und/oder regenerativen Mittels, insbesondere eines Immunregulators, Immunregenerativums, Antiinfektivums, Antimykotikums, Antitoxinozidikums Antiresistenzmittels oder Zytostatikums, zur Behandlung von Mensch und Tier;
(4) ein Verfahren zur Behandlung von unspezifischer Genese, Allergien, Krebserkrankungen (auch kombinatorisch mit bekannten Therapien wie z. B. Chemotherapien u. ä.), Symptome, die ursächlich nicht mit regenerativ therapierbaren Krankheitsbiledern assoziiert werden (wie z. B. Depressionen, Rheuma, Arthritis, vegetative Symptome, Übergewicht, Bronchialerkrankungen usw.), von Immunerkrankungen und infektiösen Erkrankungen bei Mensch und Tier umfassend Verabreichen einer Zusammensetzung aus Mikroorganismuskomponente (a) und Modulatorkomponente (b), wie vorstehend in (1) definiert; und

(5) ein Nahrungsmittel, Nahrungsergänzungsmittel, Tiernahrungsmittel oder Tier-nahrungsergänzungsmittel, umfassend eine Zusammensetzung aus Mikroorganismuskomponente (a) und Modulatorkomponente (b), wie vorstehend unter (1) definiert.

Die vorstehend unter (1) bis (2) definierten Arzneimittel können als regenerative Mittel zum Erhalten bzw. zur Wachstumsunterstützung oder -förderung von lebensnotwendigen Mikroorganismen (z. B. im Gastrointestinalertrakt, Dünndarm, Mundschleimhaut, Genitalschleimhaut) eingesetzt werden, sowohl

(A) als Mittel mit genereller regenerativer Wirkung und/oder
(B) in Multi-Step-Anwendungen (z.B. als Schritt 2 nach einer dekontaminativer Anwendung (z.B. mit einer solchen wie in der EP 00124497 beschriebenen) in Schritt 1) und/oder
(C) kombinatorisch mit bekannten Arzneimitteln, Nahrungsmitteln, Futtermitteln oder Ergänzungsstoffen.

Der Einsatz dieser Mittel (A), (B) und (C) als Regenerativum findet in/am menschlichen oder tierischen Körper statt und kann einzeln kombiniert oder im Multi-Step-Verfahren angewendet werden. Es ist fest, flüssig oder gasförmig. Bei oraler Anwendung kann es mit Zusatz-/Träger-/Füll-/Ergänzungsstoffen inerter Art ergänzt werden, sodass es in den entsprechenden Passagen von Magen, Dünn- und Dickdarm oder Ileum seine gezielte Wirkung u. ä. auch in Form von damit kombinierten Timern, funktionsinerten Stoffen mit zur gezielten zeitlich fixierten Anwendung seine Wirkung entfaltet. Andere Anwendungen sind: i. m., i. v., inhalativ, oral, intravenös, intramuskulär, rektal, Kontaktpräparat, innerlich/äußerlich (auch Schleimhaut), intraperitoneal, subkutan, auf/in inneren Organen (z. B. endoskopisch) in Form von Tabletten, flüssig, Gas, Pulver, Injektion, Infusion, Suppositorium, Spray, Salben, Pflaster.

Durch das erfindungsgemäße Arzneimittel (1) und (2) bzw. das Mittel (4) wird dem Körper Mikroorganismen (z. B. Lactobacillen, Bac. subtilis, positive Coliforme u. ä.) regenerativ zugeführt, die dann in ihrer Körperzellenadaption und in ihrer Funktion erhalten, stabilisiert oder verstärkt und vermehrt werden, sodass eine für den Körper notwendige regenerative Wirkung möglich ist, bei ca. 40 Billionen Körperzellen, jedoch 100 Billionen Mikroorganismen (ca. 400 - 500 Spezies) durchaus ein plausibler und notwendiger Vorgang, zumal durch Lebensweise und Medikation der Neuzeit das Gleichgewicht der im Köper befindlichen notwendigen Mikroorganismenspezies und deren Quantität und Qualität (Resistenzen bzw. Selektionismus, d. h. andere Keime überwuchern bzw. nehmen überhand) bei vielen Menschen und Tieren bereits gestört ist, und Ursache für viele Krankheitsbilder sind, wie vorne beschrieben.

## Detaillierte Beschreibung der Erfindung

Mikroorganismen im Sinne der vorliegenden Erfindung umfasst frische (vitale) Zellen mit oder ohne Substrate, Lysate, abgetötete Zellen, gefrorene, tiefgefrorene gekühlte, konservierte usw. Mikroorganismen, jeweils mit oder ohne Träger- oder Trennstoffe.

Dabei ist der apathogene Mikroorganismus (a1) vorzugsweise ein apathogener Lactobacillus ist und vorzugsweise ausgewählt ist aus Lactobacillus acidophilus, Lactobacillus bifidum, Lactobacillus bulgaris, Lactobacillus casei, Lactobacillus bifido longum und Lactobacillus fermentum und/oder ein Mikroorganismus, ausgewählt aus Pilzen, Dermatophyten, Hefe- und Sprosspilzen, die keine pathogenen oder toxinogenen Eigenschaften besitzen oder im Gastrointestinaltrakt vorkommen, einschließlich Sarcina, Staphylococcus epidermis, Gaffkya, Streptococcus K-P, Corynebacterium xerosis, Corynebacterium hoffmanni, Bacillus subtilis und Saccharomyces cervisiae. Die pathogenen oder fakultativ pathogenen Mikroorganismen (a2) sind vorzugsweise Serratia marcescens, Alcaligenes faecalis, Citrobacter, Hafnia alvei, Afflya tetragena, Neisseria catarrhalis, Neisseria sicca, Neisseria Flavescens-, subflava-, flava-, perflva, Veillonella parvula, Borrelia buccalis, Rhodtorula rubra, Cladosporium, Geotrichum mucor, Mucor und Torulopsis.

Neben der eindeutig als apathogen, pathogen bzw. als fakultativ pathogen klassifizierbaren Organismen können auch solche Mikroorganismen eingesetzt werden, die momentan keiner dieser Klassifizierungen zugeordnet werden können, bzw. je nach Organismus pathogen oder apathogen sind. Es handelt sich dabei zum Beispiel um bestimmte Lactobacillacae (a3), bestimmte Eubacteriales (a4) und bestimmte Enterbacteriaceae bzw. die große Gruppe der Hefen und Sprosspilze, Pilze, Dermatophyten und Viren.

Zu den Lactobacillacae (a3) zählt dabei Streptococcus A-T, Streptococcus viridans I-IV, Streptococcus salivarius, Peptostreptococcus, und Diplococcus pneumonia.

Zu den Eubacteriales (a4) zählen dabei Alkaligenes, Achromobacter, Flavobacterium, Escherichia, Shigella, Salmonella, Arizona, Klebsiella, Enterobacter, Serratia proteus, Proviridencia, Edwardsiella, Pasteurella, Yersinia, Francisella,Bordetella, Brucella, Haemophilius, Moraxella, Actinibacillus, Fusobacterium batrteroides, Fusobacterium spaerophorus, Streptobacillus, Staphylococcus, Micrococcus, Sarcina, Peptococcus, Meisseria, Veillonella, Streptococcus, Peptostreptococcus, Diplococcus, Corynebacterium, Listeria, Erysipelothrix, Bacillus und Clostridium.

Zu den Enterobacteriaceae (a5) zählen dabei Enterobacteriaceae, Brucellaceae, Micrococcaceae; Corynebacteriaceae, Bacillaceae, Actinomyceaceae, Treponemataceae, Mycoplasmataceae, Bartonellaceae, Achromobacteriaceae und Pseudomonadaceae.

Als Enzym (b1) im Sinne der vorliegenden Erfindung können Enzyme verwendet werden, die tierischen oder synthetischen Ursprungs sind. Besonders bevorzugt im Sinne der vorliegenden Erfindung ist, wenn das Enzym ausgewählt ist aus Chymotrypsin, Bromelain, Papain, Pepsin, Trypsin, ein Enzym aus Gruppen der Oxidoreduktasen, Dehydrogenasen, Oxygenasen, Transferasen, Hydrolasen, Esterasen, Glykosidasen, Proteasen, Lyasen, Isomerasen, Ligasen, Synthetasen, DNA-Ligasen, Amylasen, Lipasen, Phosphatasen, Leukoproteasen, Kathepsine, Oxidasen wie Katalasen und Peroxidasen, Carboxylasen ist und besonders bevorzugt eine Hydrolase oder Proteasen wie Bromelain, Trypsin, Ananain, Pepsin, Chymosin und Ficain ist.

Die GRAS(Generally Recognized As Safe)-Aromastoffe (b2) sind von der FDA-Behörde zur Verwendung in Nahrungsmitteln als gewerbesicher anerkannt (GRAS = Generally Recognized As Safe In Food). Bei den erwähnten GRAS-Aromastoffen handelt es sich um solche Verbindungen, die in FEMA/FDA GRAS Flavour Substances Lists GRAS 3-15 Nr. 2001-3905 (Stand 2000) genannt sind. In dieser Liste sind natürliche und naturidentische Aromastoffe aufgeführt, die von der amerikanischen Gesundheitsbehörde FDA zur Verwendung in Nahrungsmitteln zugelassen sind: FDA Regulation 21 CFR 172.515 für naturidentische Aromastoffe (Synthetic Flavoring Substances and Adjuvants) und FDA Regulation 21 CFR 182.20 für natürliche Aromastoffe (Natural Flavoring Substances and Adjuvants).

Die Funktion der Komponente (b2) beruht auf dem folgenden neuen Wirkungsprinzip: Die Zusammensetzung erlaubt die Penetration der Bestandteile in den Mikroorganismus, unterbindet damit dessen Vermehrung, zerstört ihn jedoch nicht. Die regenerative Aktivität erlaubt die Penetration in den Mikroorganismus und/oder die Körperzelle, um damit "gutartige" Mikroorganismen zu stabilisieren bzw. deren Stoffwechselaktivierung und/oder zu vermehren (kolonisieren) und/oder zu permeieren.

Die GRAS-Aromastoffe (b2) der vorliegenden Erfindung sind vorzugsweise ausgewählt aus (i) GRAS-Aroma-Alkoholen oder deren Derivate, (ii) GRAS-Polyphenolen, (iii) GRAS-Säuren oder deren Derivate, (iv) GRAS-Phenolen oder deren Derivate, (v) GRAS-Estern, (vi) GRAS-Terpenen, (vii) GRAS-Acetalen, (viii) GRAS-Aldehyden und (ix) GRAS-etherischen Ölen. Bevorzugt werden wenigstens zwei GRAS-Aromastoffe eingesetzt.

Im einzelnen können beispielsweise folgende GRAS-Aroma-Alkohole (i) zum Einsatz kommen:

Benzylalkohol, Acetoin (Acetylmethylcarbinol), Ethylalkohol (Ethanol), Propylalkohol (1-Propanol), iso-Propylalkohol (2-Propanol, Isopropanol), Propylenglykol, Glycerin, n-Butylalkohol (n-Propylcarbinol), iso-Butylalkohol (2-Methyl-1-propanol), Hexylalkohol (Hexanol), L-Menthol, Octylalkohol (n-Octanol), Zimtalkohol (3-Phenyl-2-propen-1-ol), α-Methylbenzylalkohol (1-Phenylethanol), Heptylalkohol (Heptanol), n-Amylalkohol (1-Pentanol), iso-Amylalkohol (3-Methyl-1-butanol), Anisalkohol (4-Methoxybenzylalkohol, p-Anisalkohol), Citronellol, n-Decylalkohol (n-Decanol), Geraniol, β-γ-Hexanol (3-Hexenol), Laurylalkohol (Dodecanol), Linalool, Nerolidol, Nonadienol (2,6-Nonadien-1-ol), Nonylalkohol (Nonanol-1), Rhodinol, Terpineol, Borneol, Clineol (Eucalyptol), Anisol, Cuminylalkohol (Cuminol), 10-Undecen-1-ol, 1-Hexadecanol. Als Derivate können sowohl natürliche oder naturidentische Derivate als auch synthetische Derivate eingesetzt werden. Geeignete Derivate sind z. B. die Ester, Ether und Carbonate der vorstehend genannten GRAS-Aroma-Alkohole. Besonders bevorzugte GRAS-Aroma-Alkohole sind Benzylalkohol, 1-Propanol, Glycerin, Propylenglycol, n-Butylalkohol, Citronellol, Hexanol, Linalool, Acetoin und deren Derivate.

Als Polyphenole (ii) können insbesondere die folgenden Polyphenole eingesetzt werden:

Brenzcatechin, Resorcin, Hydrochinon, Phloroglucin, Pyrogallol, Cyclohexan Usninsäure, Acylpolyphenole, Lignine, Anthocyane, Flavone, Catechine, Gallussäurederivate (z. B. Tannine, Gallotannin, Gerbsäuren, Gallus-Gerbsäuren), Carnosol, Carnosolsäure (einschließlich deren Derivate wie (2,5-Dihydroxyphenyl)carboxyl- und (2,5-Dihydroxyphenyl)alkylencarboxylsubstitutionen, Salze, Ester, Amide), Kaffesäure und deren Ester und Amide, Flavonoide (z. B. Flavon, Flavonol, Isoflavon, Gossypetin, Myrecetin, Robinetin, Apigenin, Morin, Taxifolin, Eriodictyol, Naringin, Rutin, Hesperidin, Troxerutin, Chrysin, Tangeritin, Luteolin, Catechine, Quercetin, Fisetin, Kaempferol, Galangin, Rotenoide, Aurone, Flavonole, Diole), Extrakte aus z. B. Camellia Primula. Weiterhin können auch deren mögliche Derivate, z. B. Salze, Säuren, Ester, Oxide und Ether verwendet werden. Das besonders bevorzugte Polyphenol ist Tannin (eine GRAS-Verbindung).

Als GRAS-Säuren (iii) können beispielsweise folgende Säuren zum Einsatz kommen:

Essigsäure, Aconitsäure, Adipinsäure, Ameisensäure, Apfelsäure (1-Hydroxybernsteinsäure), Capronsäure, Hydrozimtsäure (3-Phenyl-1-propionsäure), Pelargonsäure (Nonansäure), Milchsäure (2-Hydroxypropionsäure), Phenoxyessigsäure (Glykolsäurephenylether), Phenylessigsäure ($\alpha$-Toluolsäure), Valeriansäure (Pentansäure), iso-Valeriansäure (3-Methylbutansäure), Zimtsäure (3-Phenylpropensäure), Citronensäure, Mandelsäure (Hydroxyphenylessigsäure), Weisäure (2,3-Dihydroxybutandisäure; 2,3-Dihydroxybernsteinsäure), Fumarsäure, Tanninsäure und deren Derivate.

Geeignete Derivate im Sinne der vorliegenden Erfindung sind Ester (z. B. $C_{1-6}$-Alkylester und Benzylester), Amide (einschließlich N-substituierte Amide) und Salze (Alkali-, Erdalkali- und Ammoniumsalze der vorstehend genannten Säuren zu verstehen. Ebenfalls umfassen Derivate im Sinne der vorliegenden Erfindung Modifikationen der Seitenketten-Hydroxyfunktionen (z. B. Acyl- und Alkylderivate) und Modifikationen der Doppelbindungen (z. B. die perhydrierten und hydroxilierten Derivate der genannten Säuren).

Als GRAS-Phenole (iv) können folgende Phenolverbindungen zum Einsatz kommen:

Thymol, Methyleugenol, Acetyleugenol, Safrol, Eugenol, Isoeugenol, Anethol, Phenol, Methylchavicol (Estragol; 3-4-Methoxyphenyl-1-propen), Carvacrol, $\alpha$-Bisabolol, Fornesol, Anisol (Methoxybenzol) und Propenylguaethol (5-Prophenyl-2-ethoxaphenol) und deren Derivate. Derivate im Sinne der vorliegenden Erfindung sind Verbindungen, in denen die phenolische Hydroxylgruppe verestert oder verethert ist.

Als GRAS-Ester (v) kommen beispielsweise Allicin und die folgenden Acetate Iso-Amylacetat (3-Methyl-1-butylacetat), Benzylacetat, Benzylphenylacetat, n-Butylacetat, Cinnamylacetat (3-Phenylpropenylacetat), Citronellylacetat, Ethylacetat (Essigester), Eugenolacetat (Acetyleugenol), Geranylacetat, Hexylacetat (Hexanylethanoat), Hydrocinnamylacetat (3-Phenyl-propylacetet), Linalylacetat, Octylacetat, Phenylethylacetat, Terpinylacetat, Triacetin (Glyceryltriacetat), Kaliumacetat, Natriumacetat und Calciumacetat zum Einsatz. Weitere geeignete Ester sind die Esterderivate der vorstehend definierten Säuren (iii).

Als Terpene (vi) kommen insbesondere Campher, Limonen und $\beta$-Caryophyllen in Betracht.

Zu den verwendbaren Acetalen (vii) zählen insbesondere Acetal, Acetaldehyddibutylacetal, Acetaldehyddipropylacetal, Acetaldehydphenethylpropylacetal, Zimtaldehydethylenglycolacetal, Decanaldimethylacetal, Heptanaldimethylacetal, Heptanalglycerylacetal und Benzaldehydpropylenglykolacetal.

Als Aldehyde (viii) sind insbesondere Acetylaldehyd, Anisaldehyd, Benzaldehyd, iso-Butylaldehyd (Methyl-1-propanal), Citral, Citronellal, n-Caprinaldehyd (n-Decanal), Ethylvanillin, Fufurol, Heliotropin (Piperonal), Heptylaldehyd (Heptanal), Hexylaldehyd (Hexanal), 2-Hexenal ($\beta$-Propylacrolein), Hydrozimtaldehyd (3-Phenyl-1-propanal), Laurylaldehyd (Docdecanal), Nonylaldehyd (n-Nonanal), Octylaldehyd (n-Octanal), Phenylacetaldehyd (1-Oxo-2-phenylethan), Propionaldehyd (Propanal), Vanillin, Zimtaldehyd (3-Phenylpropenal), Perillaaldehyd und Cuminaldehyd verwendbar.

Als GRAS etherische Öle (ix) können insbesondere die im folgenden aufgeführten etherischen Öle und/oder die alkoholischen, glykolischen oder durch $CO_2$-Hochdruckverfahren erhaltenen Extrakte aus den genannten Pflanzen verwendet werden:

(ix1) Öle bzw. Extrakte mit hohem Anteil an Alkoholen: Melisse, Koriander, Kardamon, Eukalyptus;
(ix2) Öle bzw. Extrakte mit hohem Anteil an Aldehyden: Eukalyptus citriodora, Zimt, Zitrone, Lemongras, Melisse, Citronella, Limette, Orange;
(ix3) Öle bzw. Extrakte mit hohem Anteil an Phenolen: Oreganum, Thymian, Rosmarin, Orange, Nelke, Fenchel, Campher, Mandarine, Anis, Cascarille, Estragon und Piment;
(ix4) Öle bzw. Extrakte mit hohem Anteil an Acetaten: Lavendel;
(ix5) Öle bzw. Extrakte mit hohem Anteil an Estern: Senf, Zwiebel, Knoblauch;
(ix6) Öle bzw. Extrakte mit hohem Anteil an Terpenen: Pfeffer, Pomeranze, Kümmel, Dill, Zitrone, Pfefferminz, Muskatnuß.

In einer bevorzugten Ausführungsform der vorliegenden Erfindung enthält die Komponente (b2) einen oder mehrere GRAS-Aroma-Alkohole oder deren Derivate. Bevorzugt wird erfindungsgemäß der Einsatz von einem, zwei oder drei GRAS-Aroma-Alkoholen.

In einer weiteren bevorzugten Ausführungsform enthält (b2)

Benzylalkohol als notwendigen Bestandteil und gegebenenfalls

einen oder mehrere weitere GRAS-Aroma-Alkohole (i) oder deren Derivate und
eine oder mehrere Polyphenolverbindungen (ii) und/oder
eine oder mehrere GRAS-Säuren (iii) oder deren Derivate.

Dieser Ausführungsform können in (b2) weiterhin noch die vorstehend definierten GRAS-Aromastoffe (iv) bis (ix) enthalten sein. Besonders bevorzugt unter den weiteren GRAS-Aromastoffen sind die Phenole (iv), Aldehyde (viii) und etherische Öle (ix).

Besonders bevorzugt im Sinne der vorliegenden Erfindung sind solche Komponenten (b2), die ausschließlich aus GRAS-Aromastoffen bestehen, d. h. keine "Derivate" der GRAS-Aromastoffe enthalten sind. Als Beispiel einer solchen Zusammensetzung ist ein Gemisch aus Benzylalkohol, einem oder zwei der vorstehend genannten GRAS-Aroma-Alkohole (i) und Tannin zu nennen. Ein weiteres Beispiel ist ein Gemisch aus zwei Alkoholen (i), einem Polyphenol (insbesondere Tannin) und einem etherischen Öl (ix), insbesondere dem phenolischen etherischen Öl (ix3).

In einer weiteren bevorzugten Ausführungsform enthält (b2) wenigstens zwei GRAS-etherische Öle (ix). Hierbei handelt es sich vorzugsweise um die vorstehend aufgeführten etherischen Öle und/oder die alkoholischen, glykolischen oder durch $CO_2$-Hochdruckverfahren erhaltenen Extrakte (ix1) bis (ix6). Daneben können noch weitere GRAS-Aromastoffe wie Alkohole (i), Polyphenolverbindungen (ii), Säuren (iii), Phenole (iv), Ester (v), Terpene (vi), Acetale (vii), Aldehyde (viii), deren Derivate enthalten. Besonders bevorzugte Zusammensetzungen dieser Ausführungsform enthalten wenigstens drei GRAS-etherische Öle (ix) und/oder enthalten neben den etherischen Ölen (ix) die weiteren GRAS-Aromastoffe Anisol und Quercitin.

In einer weiteren bevorzugten Ausführungsform enthält (b2) wenigstens einen lipophilen und wenigstens einen hydrophilen GRAS-Aromastoff (es ist hier jedoch anzuführen, das auch hydrophil-hyrophil und lipophil-lipophil GRAS-Aromastoff-Kombinationen für die erfindungsgemäßen Arzneimittel geeignet sind). Der hydrophile GRAS-Aromastoff kann dabei ein hydrophiler, alkoholischer GRAS-Aromastoff ($i_h$) und/oder ein hydrophiler, nichtalkoholischer GRAS-Aromastoff sein. Dabei sind solche Zusammensetzungen bevorzugt, die neben den genannten hydropilen Verbindungen weiterhin Benzylalkohol und/oder eine Polyphenolverbindung (ii) enthalten.

Hydrophile, alkoholische GRAS-Aromastoffe ($i_h$) sind dabei einwertige oder mehrwertige Alkohole mit 2 - 10 C-Atomen, vorzugsweise mit 2 - 7 C-Atomen. Besonders bevorzugte Verbindungen sind 1-Propanol, Glycerin, Propylenglykol und Acetoin. Hydrophile, nichtalkoholische GRAS-Aromastoffe sind ausgewählt aus organischen Säuren ($iii_h$) mit 1 - 15 C-Atomen und physiologisch akzeptablen Salzen derselben, hydrophilen Acetaten ($v_h$) und hydrophilen Aldehyden ($viii_h$). Bevorzugte organische Säuren ($iii_h$) sind solche mit 2 - 10 C-Atomen und insbesondere Essigsäure, Acronitsäure, Ameisensäure, Apfelsäure, Milchsäure, Phenylessigsäure, Zitronensäure, Mandelsäure, Weinsäure, Fumarsäure, Tanninsäure, Hydrozimtsäure und deren physiologisch akzeptablen Salze. Das hydrophile Acetat ($v_h$) ist vorzugsweise ausgewählt aus Allicin, Triacetin, Kaliumacetat, Natriumacetat und Calciumacetat und der hydrophile Aldehyd ($viii_h$) ist vorzugsweise ausgewählt aus Furfurol, Propionaldehyd und Vanillin.

In dieser Ausführungsform sind die lipophilen GRAS-Aromastoffe vorzugsweise ausgewählt aus ($i_l$) lipophilen GRAS-Aroma-Alkoholen oder deren Derivate, (ii) Polyphenolverbindungen, ($iii_l$) lipophilen GRAS-Aromasäuren oder deren Derivate, (iv) Phenolen oder deren Derivate, ($v_l$) lipophilen Estern, (vi) Terpenen, (vii) Acetalen, ($viii_l$) lipophilen Aldehyden und (ix) etherischen Ölen. Bevorzugt in dieser Ausführungsform ist, dass (c5) zwei der genannten lipophilen GRAS-Aromastoffe enthält.

Geeignete lipophile GRAS-Aroma-Alkohole ($i_l$) aus den vorstehend definierten Alkoholen (i) sind dabei insbesondere:

aromatischen GRAS-Aroma-Alkoholen, umfassend Benzylalkohol, 2-Phenylethanol, 1-Phenylethanol, Zimtalkohol, Hydrozimtalkohol, 1-Phenyl-1-Propanol und Anisalkohol und aliphatischen GRAS-Aroma-Alkoholen, umfassend n-Butylalkohol, iso-Butylalkohol, Hexylalkohol, L-Menthol, Octylalkohol, Heptylalkohol, n-Amylalkohol, iso-Amylalkohol, Anisalkohol, Citronellol, n-Decylalkohol, Geraniol, β-γ-Hexanol, Laurylalkohol, Linalool, Nerolidol, Nonadienol, Nonylalkohol, Rhodinol, Terpineol, Borneol, Clineol, Anisol, Cuminylalkohol, 10-Undecen-1-ol und 1-Hexadecanol und deren Derivate. Hierbei sind die aromatischen GRAS-Aroma-Alkohole und insbesondere Benzylalkohol bevorzugt.

Die lipophile Polyphenolverbindung (ii), Phenole oder deren Derivate (iv), Terpene (vi), Acetale (vii) und etherischen Öle (ix) sind dabei bevorzugt die vorstehend definierten Verbindungen (ii), (iv), (vi), (vii) und (ix). Die lipophilen GRAS-Aromasäuren oder deren Derivate ($iii_l$), lipophilen Ester ($v_l$) und lipophilen Aldehyde umfassen alle spezifisch genannten Säuren, Ester und Aldehyde mit Ausnahme der vorstehend spezifisch genannten Verbindungen ($iii_h$), ($v_h$) und ($viii_h$).

In einer besonders bevorzugten Ausführungsform enthält (b2) entweder

- zwei lipophile GRAS-Aroma-Alkohole ($i_l$), jedoch kein Benzylalkohol und keine Polyphenolverbindungen (ii) oder
- Benzylalkohol und/oder eine Polyphenolverbindung (ii), jedoch keine weiteren GRAS-Aroma-Alkohole.
  Hierbei ist besonders bevorzugt, wenn (b2) ausschließlich nichtalkoholische, hydrophile GRAS-Aromastoffe, insbesondere ausschließlich eine hydrophile GRAS-Aromasäure ($iii_h$) enthält.

In einer weiteren besonders bevorzugten Ausführungsform enthält (b2)

- einen oder mehrere GRAS-Aroma-Alkohole ($i_l$) oder deren Derivate und
- einen oder mehrere Aromastoffe, ausgewählt aus Polyphenolverbindungen (ii) und lipophilen GRAS-Aromasäuren oder deren Derivate ($iii_l$).
Daneben kann (b2) weitere GRAS-Aromastoffe, ausgewählt aus (iv) Phenolen oder deren Derivate, ($v_l$) lipophilen Estern, (vi) Terpenen, (vii) Acetalen, ($viii_l$) lipophilen Aldehyden und (ix) etherischen Ölen enthalten. Weiterhin ist bevorzugt, wenn in dieser besonders bevorzugten Ausführungsform von (b2) Benzylalkohol als notwendigen Bestandteil und gegebenenfalls einen oder mehrere weitere lipophile GRAS-Aroma-Alkohole oder deren Derivate ($i_l$) enthalten ist. Auch können hierbei weitere lipophile GRAS-Aromastoffe (iv) - (ix), wie vorstehend definiert, enthalten sein. Diese weiteren lipophilen GRAS-Aromastoffe sind besonders bevorzugt Phenole (iv) und/oder etherische Öle (ix).

In einer weiteren besonders bevorzugten Ausführungsform enthält (b2) zwei lipophilen GRAS-Aroma-Alkohole und wenigstens eine Polyphenolverbindung (ii), die dabei bevorzugt Tannin ist.

Besonders bevorzugt im Sinne der vorliegenden Erfindung sind dabei solche Zusammensetzungen (b2), die ausschließlich aus GRAS-Aromastoffen bestehen, d. h. keine "Derivate" der GRAS-Aromastoffe enthalten. Als Beispiel einer solchen Zusammensetzung ist ein Gemisch aus Benzylalkohol, einem oder zwei der vorstehend genannten GRAS-Aroma-Alkoholen ($a_l$) und Tannin zu nennen. Dieses Gemisch enthält dabei vorzugsweise 0.1 bis 98 Gew.-% Benzylalkohol und 0.01 - 10 Gew.-%, vorzugsweise 1 - 10 Gew.-%, Tannin. Ein weiteres Beispiel einer bevorzugten Zusammensetzung ist ein Gemisch aus 2 Alkoholen, einem Polyphenol (insbesondere Tannin) und einem etherischen Öl (insbesondere einem phenolischen etherischen Öl, Komponente (i3)).

In einer weiteren besonderen Ausführungsform der vorliegenden Erfindung enthält (b2) wenigstens eine GRAS-Aromastoff mit Doppelbindung ($\Delta$) oder ein Derivat davon, vorzugsweise wenigstens zwei solcher Verbindungen. Als Beispiele der Verbindung ($\Delta$) sind dabei ungesättigte GRAS-Alkohole ($i_\Delta$) wie Zimtalkohol, Cironellol, 3-Hexenol, Nonadienol und 10-Undecen-1-ol, ungesättigte GRAS-Säuren ($iii_\Delta$) wie Zimtsäure und Fumarsäure, ungesättigte GRAS-Ester ($iv_\Delta$) wie Zimtsäureester (z.B. Ethylcinnamat und Propylcinnamat), Cinnamylacetat und Citronellylacetat, ungesättigte GRAS-Acetale ($vii_\Delta$) wie Zimtaldehydethylenglycolacetal und ungesättigte GRAS-Aldehyde ($viii_\Delta$) wie Zimtaldehyd und Citronellal. Als weitere GRAS-Aromastoffe enthalten diese Zusammensetzungen vorzugsweise etherische Öle (ix) und/oder die vorstehend definierten hydrophilen GRAS-Aromastoffe.

Nachfolgend sind besonders bevorzugte Zusammensetzungen (b2) aufgeführt. Die Mengenangaben, die - falls nicht anders aufgeführt - in Gew.-% angegeben sind, sind nur besonders bevorzugte Ausführungsformen der jeweiligen Zusammensetzungen.

1. Zusammensetzungen mit wenigstens zwei GRAS-Aromastoffen oder deren Derivaten:

45% Anisalkohol (i), 35% Borneol (i), 20% Rhodinol (i) ;
82% Valeriansäure (iii), 18% Campher (ix3);
10% Apfelsäure (iii), 90% Acetoin (i);
12% $\alpha$-Bisabolol (iv), 18% Geranylacetat (i), 20% Rhodinol (i).

2. Zusammensetzungen mit einem oder mehreren GRAS-Aroma-Alkoholen

(a) und einem oder mehreren GRAS-Aromen:

65% Propylenglykol (i), 10% Kaffeesäure (ii), 10% Tannin (ii), 5% Resveratrol (ii), 10% Milchsäure (iii);
70% Propylenglycol (i), 20% Anisalkohol (i), 5% Quercetin (ii), 5% Tannin (ii);
82% L-Menthol (i), 8% Anisol (iv), 7% Citronellol (i), 3% Safrol (iv);
98% Propylenglycol (i), 2% Allicin (v).

3. Zusammensetzungen mit dem notwendigen Bestandteil Benzylalkohol:

79% Benzylalkohol (i), 20% Geraniol (i), 1% Tannin (ii);
95% Benzylalkohol (i), 5% Mandelsäure (iii);
70% Benzylalkohol (i), 30% Catechin (ii);
1% Benzylalkohol (i), 88% Propylenglycol (i), 1% Tannin (ii), 10% Milchsäure (iii).

4. Zusammensetzungen mit wenigstens 2 GRAS-etherischen Ölen (i)

90% Pomeranze (ix6), 10% Zimt (ix2);
6% Oreganum (ix3), 8% Koriander (ix1), 7% Citronensäure (iii), 79% Propylenglycol (Trägerstoff);
1% Lavendel (ix4), 1% Anis (ix3), 2% Safrol (iv), 96% Xanthan (Trägerstoff);
5% Kardamon (ix1), 5% Anis (ix3), 10% Eukalyptus (ix2), 80% Trägerstoffe, insbesondere Alginsäure.

5. Zusammensetzung mit wenigstens einem lipophilen und wenigstens einem hydrophilen GRAS-Aromastoff:

25% Zimtalkohol ($i_l$), 25% Linalool ($i_l$), 50% Glycerin ($i_h$);
25% Zimtalkohol ($i_l$), 25% Linalool ($i_l$), 50% Milchsäure ($iii_h$);
50% Benzylalkohol ($i_l$), 50% Glycerin ($i_h$);
50% Benzylalkohol ($i_l$), 50% Milchsäure ($iii_h$);
50% Tannin (ii), 50% Glycerin ($i_h$);
50% Tannin (ii), 50% Milchsäure ($iii_h$);
45% Zimtalkohol ($i_l$), 40% Linalool ($i_l$), 10% Tannin (ii), 5% Vanillin ($viii_h$).

6. Zusammensetzungen mit wenigstens einem GRAS-Aromastoff mit Doppelbindung:

70% Zimtaldehyd ($viii_\Delta$), 30% Oreganum (ix3);
20% Eugenol (iv), 50% Citronellol ($i_\Delta$), 20% Citronellal ($viii_\Delta$), 10% Ethylcinnamat ($v_\Delta$);
10% Zimtaldehyd ($viii_\Delta$), 90% Glycerin (i).

Die tierischen und pflanzlichen Extrakte (b3) enthalten wenigstens eine Komponente mit alkoholischer, Säure-, Ester, Aldehyd-, Acetal-, phenolischer, funktionellen Gruppen und/oder mit Doppelbindungen. Hierbei wird insbesondere bevorzugt phenolische Säure, alkoholische, aldehydische Doppelbindungen. Besonders bevorzugt ist dabei, wenn die Extrakte folgenden Ursprungs sind bzw. die folgenden Komponenten enthalten:

Aloe, Annatto (Samen), Arnika, Baldrian, Beinwell, Birkenblätter, Blütenpollen, Brennessel, Champignon, Dill, Efeu, Eibisch, Eichen(rinde), Fichten(nadeln), Gingko(blätter), Ginseng(wurzel), Hamamelis(blätter) Henna, Heublumen, Hibiskus, Hopfen, Huflattich, Ingwer(wurzel), Johanniskraut, Kakao, Kaffee, Kamille, Karotten, Knoblauch, Kolanuss, Koriander, Kümmel, Lavendel, Linden(blüten), Löwenzahn, Malven, Mäusedorn, Majoran, Melisse, Orangen(schalen), Orthosiphonis, Paprika, Pfeffer, Pfefferminze, Pilze, Rhabarber, Ringelblumen, Rosmarin, Rosskastanien, Salbei, Schachtelhalm, Schöllkraut, Schwarztee, Süssholz, Wacholder(beeren), Weißdorn, Weizenkleie, Zimt, Algen, Banane, Benzo, Birkenteer, Bochshorn, Boldo, Chinarinde, Copai-Balsam, Derriswurzel, Kajeputöl, Enzian, Eukalyptus, Fenchel, Fieberklee, Kalmus, Kartoffelstärke, Kiefer, Kolophonium, Zwiebel, Lärchenterpentin, Leinsamen, Lorbeer, Nadelholzteer, Niduli-Baum, Perubalsam, Pyrethrum, Quassiaholz, Reis, Rotenon, Sadebaum, Saponin, Schwarzwurzel, Senf, Sesam, Soja, Sonnenhut, Tausendgüldenkraut, Terpentinöl, Wegerich, weiße Nieswurz, Wermut, Zypresse, Apfel, Avocado, Theobroma grandiflorum, Grapefruit, Guarana, Gurke, Hafer, Melone, Jojoba, Kiwi, Klette, Klee, Kokosnuss, Kornblume, Mandel, Mango, Mistel, Taxus, Papaya, Passionsfrucht, Pfirsich, Schafgarbe, Schlüsselblume, Sonnenblume, Taubnessel, Thymian, Walnuss, Wasserminze, Ylang Ylang, Cochenille, Bienenwachs, Gelee Royal, Propolis, Schelllack, Lecithin, Formiate, Ameisensäure, Lutein, Canthaxanthin, Riboflavin, Lactoflavin, Speisefettsäuren und -derivate, Angora, Astrahan, Cashmere, Mohair, Wolle, Milch, Kasein, Kaseinogen, Bienenpollen, Bienengift, Honig, Molke, Eier, Karatin, Plazenta, Fisch, Fleisch, Geflügel, Ambra, Capiz, Kaviar, Chitin, Koralle, Squalene, Seehundöl, Walöl, Schwämme, Perlen, Rogen, Robbe, Schalentiere, Spermöl, Samenöl, Wachs, Karmine, Karminsäure, Catgut, Moschus, Zibet, Rizinus, Östrogen, Progesteron, Testeron, Hormone, Elfenbein, Lanolin, Nerzöl, Pergament, Seide, Schlangengift, Harnstoff, Aminosäuren, Aspik, Knochen, Borsten, Kollagen, Daunen, Blut, Gelatine, Glycerin, Fell, Leder, Oleostearin, Lab, Stearinderivate.

Neben den Komponenten (a) und (b) kann das Arzneimittel weiterhin eine Zusatzstoffkomponente (c) enthalten, die ausgewählt ist aus

(c1) wenigstens einem Vitamin,
(c2) wenigstens einem Mineralstoff oder Spurenelement,
(c3) wenigstens einem Wachstumsförderer oder Supporter,
(c4) wenigstens einem Zusatzstoff insbesondere Trägerstoff, Trennmittel oder Timersubstanz,
(c5) wenigstens einem Enzymzusatzstoff,

Geeignete Vitamine (c1) im Sinne der vorliegenden Erfindung sind insbesondere Ascorbinsäure, Ascorbinpalmitat, Ascorbate, insbesondere Magnesiumascorbate, β-, γ- und δ-Tocopherol, α- und β-Carotin, Thiamin, Riboflavin, Niacin, Pantothensäure, Calciumpantothenat, Pyridoxinhydrochlorid, Cyanocobalamin, Col-Calciferol-Cholerterin, Biotin, Folsäure und Nicotinamid. Geeignete Mineralstoffe (c2) sind insbesondere Calcium, Phosphor, Eisen, Magnesium, Iod, Kalium, Chlor, Kupfer, Mangan, Chrom, Molybdän, Selen, Silicium und Zinn. Wachstumsförderer und Supporter (c3) im Sinne der vorliegenden Erfindung umfassen Hefe, Zucker (einschließlich Milch-, Trauben-, Rüben- und Rohrzucker), Gelee Royale, Pepton, Gallensalze, Kochsalz, Agar, Gelatine, Kartoffelextrakt, Fleischextrakt, Citrate, Rinderherzgewebe, Stärke, Nikotinsäure, Tryptose, Natriumazid, Mannit, Magnesiumsulfat, Dinatriumhydrogenphosphat, Kaliumhydrognphosphat, NatriumIpyruvat, Caseinpepton, Glycin, Lithiumchlorid, Dikaliumphosphat, Natriumsulfit, Eisensulfat, Proteose-Pepton, Leber-Verdauungprodukt, Kartoffelinfus, Ochsengalle, Natriumthioglycolat, Calciumchlorid, Sojamehl, Sojamehl-Pepton, L-Cystein, L-Asparagin, Natriumdithionit, Lecithin, Mineralstoffe und deren Salze, Rinder- und Schafsserum, Desoxyribonukleinsäuren, Acetate, Adeninsulfat, Guaninhydrochlorid, Caseinhydrolysat, Kalbshirninfusion, Rinderherzinfusion, Holzkohle, Leberinfusion. Die Zusatzstoffe (c4) der vorliegenden Erfindung sind vorzugsweise ausgewählt aus Salzen, Zucker, Kohlenhydraten, Eiweiß, Proteinen, Flüssigkeiten, insbesondere Ölen und Wasser, Fette, Fettsäuren, und Trägerstoffen, insbesondere Propylenglycol, Benzylalkohol, Glycerin, Alginate, Lactate, Kieselsäure, Gelatine und Agar. Geeignete Enzymzusatzstoffe (c5) sind insbesondere Inisitol, Rutin, Lutein, L-Arginin, L-Cystein, L-Karnitin, L-Lysin, L-Prolin und Coenzym Q10.

Bevorzugte Zusammensetzungen sind dabei die Zusammensetzungen (I) bis (VI), die die folgenden Komponenten enthalten:

(I) (a) wenigstens einen apathogenen Mikroorganismus (a1), vorzugsweise Lactobacillus acidophilus und/oder Lactobacillus fermentum, (b) wenigstens ein Enzym (b1), vorzugsweise eine Hydrolase und/oder Protease, und gegebenenfalls noch eine Zusatzstoffkomponente (c), ausgewählt aus Vitaminen (c1), Mineralstoffen und Spurenelementen (c2);

(II) (a) wenigstens einen apathogenen Mikroorganismus (a1), vorzugsweise Lactobacillus acidophilus und/oder Lactobacillus fermentum, (b) wenigstens einen GRAS-Aromastoff oder ein Derivat davon (b2), vorzugsweise Kardamon, Anis und/oder Tannin, und gegebenenfalls noch eine Zusatzstoffkomponente (c), ausgewählt aus Vitaminen (c1), Mineralstoffen und Spurenelementen (c2), und/oder ein oder mehrere Enzyme (b1);

(III) (a) wenigstens einen apathogenen Mikroorganismus (a1), vorzugsweise Lactobacillus acidophilus und/oder Lactobacillus fermentum, (b) wenigstens einem tierischen oder pflanzlichen Extrakt oder ein Derivat davon (b3), vorzugsweise ein Extrakt von Taxus, Gingko, Avocado und/oder Bienenpollen, und gegebenenfalls noch eine Zusatzstoffkomponente (c), ausgewählt aus Vitaminen (c1), Mineralstoffen und Spurenelementen (c2), ein oder mehrere Enzyme (b1) und/oder einen oder mehrere GRAS-Aromastoffe oder ein Derivat davon (b2);

(IV) (a) wenigstens einen pathogenen oder fakultativ pathogenen Mikroorganismus (a2), vorzugsweise Bacillus subtilis, Serratia marcescens und/oder Streptococcus, (b) wenigstens ein Enzym (b1), vorzugsweise eine Hydrolase und/oder Protease, und gegebenenfalls noch eine Zusatzstoffkomponente (c), ausgewählt aus Vitaminen (c1), Mineralstoffen und Spurenelementen (c2) und/oder einen apathogenen Mikroorganismus (a1);

(V) (a) wenigstens einen pathogenen oder fakultativ pathogenen Mikroorganismus (a2), vorzugsweise Bacillus subtilis, Serratia marcescens und/oder Streptococcus, (b) wenigstens einen GRAS-Aromastoff oder ein Derivat davon (b2), vorzugsweise Kardamon, Anis und/oder Tannin, und gegebenenfalls noch eine Zusatzstoffkomponente (c), ausgewählt aus Vitaminen (c1), Mineralstoffen und Spurenelementen (c2), und/oder ein oder mehrere Enzyme (b1) und/oder einen apathogenen Mikroorganismus (a1);

(VI) (a) wenigstens einen pathogenen oder fakultativ pathogenen Mikroorganismus (a2), vorzugsweise Bacillus subtilis, Serratia marcescens und/oder Streptococcus, (b) wenigstens einem tierischen oder pflanzlichen Extrakt oder ein Derivat davon (b3), vorzugsweise ein Extrakt von Taxus, Gingko, Avocado und/oder Bienenpollen, und gegebenenfalls noch eine Zusatzstoffkomponente (c), ausgewählt aus Vitaminen (c1), Mineralstoffen und Spurenelementen (c2), ein oder mehrere Enzyme (b1) und/oder einen oder mehrere GRAS-Aromastoffe oder Derivate davon (b2) und/oder einen apathogenen Mikroorganismus (a1).

Am meisten bevorzugte Zusammensetzungen sind dabei solche Zusammensetzungen, die der in dem experimentellen Teil der Anmeldung genannten Zusammensetzung BHQ R entsprechen (wobei sie nicht auf den speziellen Gehalt der Komponente beschränkt sind).

Neben den Komponenten (a) bis (c) können in den erfindungsgemässen Arzneimitteln (1) und Nahrungsergänzungsmitteln (5) noch weitere handelsübliche pharmakologisch akzeptable Verbindungen und Trägermaterialien (k) wie Alkohole (k1) Emulgatoren (k2), Stabilisatoren (k3), Antioxidantien (k4), Konservierungsmittel (k5), Lösemittel (k6), Salben (k7), Trägerstoffe (k8) einschließlich solcher mit "Timer"-Funktion (d. h., Auflösung = Wirkung an der Stelle der Bestimmung), etc. vorhanden sein. Der Anteil der Komponenten (k) an dem Arzneimittel bzw. Mittel richtet sich dabei nach der Applikationsform des Arzneimittels/Mittels und darf bis zu 95 Gew.-% betragen, ist vorzugsweise kleiner als 10 Gew.-% und liegt bevorzugt im Bereich von 0,1 bis 5 Gew.-%. So ist die Menge der Zusatzstoffe sehr gering bei Inhalationsmitteln (Aerosolen) mit einem Gehalt von mikrobizider Zusammensetzung von bis zu über 90 Gew.-% des Aerosols, ist jedoch deutlich größer z. B. bei oraler, intravenöser oder intramuskulärer Applikation des Arzeinmittels, bei der der Gehalt an mikrobizider Zusammensetzung gewöhnlich im Bereich von 0,1 bis 20 Gew.-% liegt, jedoch kann er auch in Anwendungen bis 95, ja sogar bei 100 Gew.% der funktionellen Zusammensetzung sein. Ebenso ist das Verhältnis der Verbindungen (k) untereinander abhängig von der Applikationsform des Arzneimittels.

Bei den Alkoholen (k1) handelt es sich erfindungsgemäß um einwertige oder mehrwertige Alkohole mit 2 bis 10 C-Atomen, vorzugsweise mit 2 bis 7 C-Atomen, wobei die GRAS-Alkohole (a) hiervon nicht umfasst sind. Vorzugsweise werden solche Mengen an GRAS-Aroma-Alkoholen (a) und weiteren Alkoholen (k1) eingesetzt, daß deren Mischungsverhältnis zwischen 1000 : 1 und 1 : 1000, insbesondere zwischen 100 : 1 und 1 : 100 und besonders bevorzugt zwischen 10 : 1 und 1 : 10 liegt.

Das Arzneimittel kann dabei in fester, flüssiger oder gasförmiger Form für Mensch und Tier verabreichbar vorliegen. Das Arzneimittel kann ein inhalatives, orales, intravenöses intramuskuläres, rektales Mittel, Kontaktpräparat, innerlich/äußerliches (auch Schleimhaut), intraperitoneales, subkutanes Mittel sein, auf/in inneren Organen (z. B. endoskopisch) in Form von Tabletten, flüssig, Gas, Pulver, Injektion, Infusion, Suppositorium, Spray, Salben, Pflaster, seine Wirkung entfalten. Das Arzneimittel kann dabei präventiv als auch zur Behandlung des akuten Befalls verabreichbar sein.

Gemäß Ausführungsform (3) betrifft die Erfindung weiterhin die Verwendung der vorstehend definierten mikroziden Zusammensetzung (1) zur Herstellung von Mitteln zur Behandlung von Mensch und Tier, insbesondere Bronchitis, Pneumonie (inhalativ).

Weiterhin betrifft die Erfindung Verfahren zur Behandlung von Immunerkrankungen und infektiösen Erkrankungen von Mensch und Tier (4), z.B. die Behandlung von Gastritis, Colitis, Ulcerosa, etc.

Die benötigte Dosis richtet sich dabei nach Art und Schwere der Krankheit, Alter, Geschlecht, Gewicht und allgemeinen Gesundheitszustand des Patienten und liegt gewöhnlich im Bereich von 0,1 bis 1000 mg, vorzugsweise 1 bis 10 mg pro kg Körpergewicht des Patienten pro Tag.

Die Behandlung kann dabei gemäß der folgenden Behandlungsschemata I-III erfolgen:

I. Multi-Step:

1. "Dekontaminativum" zum Entkeimen krankheits- oder infektionsverursachender Erreger im/am Körper mit aromahaltigen Arzneimitteln (rezeptur- und dosierabhängig)
2.

(a) "Regenerativum" zum Regenerieren (Erhalten oder Wachstumsförderung) notwendiger Mikroorganismen im/am Körper mit aromahaltigen Arzneimitteln (rezeptur- und dosierabhängig)
(b) eventuelle Zugabe gleichzeitig oder danach von "positiv notwendigen" Mikroorganismen (z. B. symbiontisches Regenerativum mit Mikroorganismen (einzeln oder Gemische), und/oder Extrakte aus Pflanzen und Tieren und/oder Vitaminen (einzeln oder Gemische), und/oder Mineralien (einzeln oder Gemische) und/oder Enzymen (einzeln oder Gemische); jedoch rezeptur- und dosierabhängig)

II. Kombinatorisches Verfahren:

1. + 2. (a) +(b) gleichzeitig

1. + 2. (a)

1. + 2. (b)

2. (a) + 2. (b)

III. Einzelanwendung: 1, 2a, 2b und/oder kombinatorisch oder Multi-Step mit Arzneimitteln des Standes der Technik (z. B. Antiinfektiva, Antidepressiva, Zytostatika, Kontrazeptiva u. v. a. m.)

Die Nahrungsergänzungsmittel und Tiernahrungsmittel/-ergänzungs-mittel (5) der vorliegenden Erfindung weisen vorzugsweise 0,1 bis 20 Gew.-% der Zusammensetzung aus Komponente (a), (b) und (c) auf, jedoch kann er auch in Anwendungen bis 95, ja sogar bei 100 Gew.-% der funktionellen Zusammensetzung liegen.

Die vorliegende Erfindung wird anhand der nachfolgenden Beispiele näher erläutert.

**Beispiele**

Materialien und Methoden

Die Mengenangaben in den nachfolgenden Rezepturen sind, falls nicht anders angegeben, in Gew.-% angegeben.

BHQ R11: Mikroorganismen (apath./fak. path./path.), Enzyme, Vitamine Mineralstoffe

Inhibition: gastrointestinale Erkrankungen (Diarrhoe, Colitis u. ä.)
Applikation: oral (1,5 g/Tablette)
Lactobacillus Bifidus $10^{11}$, Lactobacillus acidophilus $10^{12}$, Bacillus subtilis $10^8$, E. coli $10^4$, Enzyme: L-Prolin 3,5 %, L-Lysin 3,5 %, L-Karnitin 1,2 %, L-Arginin 1,5 %, Trysin 3,0 %, Bromelain 1,8 %, Vitamine: C 20 %, E 2,5 %, A 15 % , B1, B2, B3, B5, B6, B12, Mineralien: Calcium, Chrom, Kupfer, Mangan, Magnesium, Selen, Zink, Phosphor ad 100 % Trägerstoffe

BHQ R8: Mikroorganismen (apathogen), Aromasynerg., etherische Öle Indikation: Atemwegserkrankungen (Bronchitis, Pneumonie u. ä.) Applikation: inhalativ
Lactobacillus bifidus, $10^{13}$, Lactobacillus acidophilus $10^{12}$, Aromen (etherische Öle): 1 % Oreganum, 0,5 % Thymian, 0,1 % Kampfer, 0,5 % Anis, 1 % Estragon, 2 % Muskat, 0,1 % Melisse, 1 % Kardamon, 1 % Fenchel, 5 % Benzylalkohol, ad 100 % Propylenglykol

BHQ R15: Mikroorganismen (apath./fak. path./path.) Aromasynerg., Enzyme, Pflanzenextrakte, Vitamine, Mineralstoffe

Indikation: mögliche Tumorizide/zytostatische Wirkung bei Tumor- bzw. Krebserkrankungen
Applikation: oral
Lactobacillus acidophilus $10^{14}$, Bacillus subtilis $10^8$, Enterococcus d. $10^6$, etherische Öle: 5 % Kardamon, 5 % anis, 10 % Eukalyptus citriodora, Enzyme: 1 % Amylase, 0,01 % Leukoprotease, 0,01 % Trypsin, Vitamine, Mineralstoffe, Pflanzenextrakt, 0,1% Chinarinde (60 T) Gingko (40 T), ad 100 % Propylenglykol in Trägerstoff (Tablette 20 % Wirkstoff)

BHQ R7: Mikroorganismen (apath.), Pflanzenextrakt, Vitamine, Mineralstoffe

Indikation: allergische Hauterkrankungen (Neurodermitis, Ekzeme u. ä.) Applikation: Salbe äußerlich
Lactobacillus acidophilus $10^{14}$, Bacillus subtilis $10^{10}$, Pflanzenextrakt: Aloe 3 %, Algen 5 %, Lärchenterpentin 0,1 %, Sesam 1 %, Lecithin 0,5 %, Avocado 2 %, Vitamine, Mineralstoffe, ad 100 % Salbengrundlage

BHQ R13: Mikroorganismen (apath.), Aromasynerg. (GRAS-Alkohole,

-Polyphenol), Vitamine, Mineralstoffe
Indikation: allergische Erkrankungen (Neurodermitis, Asthma u. ä.) Applikation: oral (flüssig)
Lactobacillus acidophilus $10^{16}$, Aroma: 1 % Benzylalkohol, 3 % Tannin, 10 % Milchsäure, 3 % Vitamine, 3 % Mineralstoffe, 80 % Propylenglykol

BHQ R6: Mikroorganismen (apath./fak. path), Aromasynerg., Enzyme Indikation: Depression

Applikation: oral
Lactobacillus brevis $10^{12}$, Lactobacillus acidophilus $10^{16}$, Lactobacillus bifidus $10^{12}$, Lactobacillus Bifidolongum $10^{14}$, Serratia marcescens $10^5$, Aromasynerg.: 20 % Benzylalkohol, 5 % Kardamon, 3 % Tannin, 2 % Eukalyptus citridora, Enzyme: 5 % Trypsin, 5 % Papain, 1 % Chymosin, 2 % Ficain, 2 % Ananain, 55 % Trägerstoff (30 % Wirkstoff in Trägerstoff Tablette 1,5 g)

BHQ R16: Mikroorganismen (apath.), Enzyme Vitamine

Indikation: Verdauungsinsuffizienz, Speisenunverträglichkeit, Appetitlosigkeit, unspezifische Magen-Darm-Beschwerden

Applikation: oral

Lactobacillus brevis $10^6$, Lactobacillus acidophilus $10^{15}$, Lactobacillus bifidus $10^{18}$, Lactobacillus bifidolongum $10^{14}$, Enzyme: 12 % Gesamt zu je gleichen Teilen: (15 % Amylase, Protease-Gemisch 85 % (18 verschiedene Enzyme) 3 % Bromelain, 5 % Papain, Vitamine 3 %, ad 100 % Trägerstoff (Tablette 2,5 g).

BN 3 (Vergleichsbeispiel):

3 Lactobazillen der Gruppen Acidophilus, Bifidus, Bifidolongum

13 Vitamine (A, E, C, K, B1, B2, B6, B12, D, Biotin, Folsäure, Nicotinamid, Pantothensäure)

14 Mineralstoffe/Spurenelemente (Ca, P, Fe, Mg, J, K, Cl, Cu, Mn, Cr, Mb, Se, Si, Zn)

Beispiel 1

Folgende Wirknachweise und Tests wurden aus wie folgt beschriebenen Gründen ausgeführt: Erhöhung der Wachstumsraten von Mastküken ist ein Prüfkriterium von regenerativen Arzneimitteln. Mastküken neigen in der Aufzucht und Massentierhaltung zu Krankheitsbildern wie Diarrhoe oder Bronchitis, was schließlich zu Wachstums- und/oder Gewichtsverlusten in der Aufzuchtphase führt, sodass das Gewichtsdifferenz der unbehandelten und behandelten Mastküken ein Prüfkriterium darstellt. Es ist bekannt, dass die meisten Mastküken per Futtermitteladditive und/oder Impfung antibiotisch und/oder vakzinspezifisch gegen Krankheit und damit einhergehenden Gewichtsverlust heutzutage behandelt werden müssen.

## Tabelle 1: Wirksamkeit gegen gastrointestine Erkrankungen

| Stall Mastküken Anzahl | Schlachtergebnis g/Masthänchen Behandlung 1. - 28. Tag<br><br>Futtermittelzusatz | | Mehrgewicht durch BHQ-Behandlung |
|---|---|---|---|
| | unbehandelt | 40 mg BHQ R11/kg | |
| 12000 Stall un-behandelt | durchschnittl. Schlachtgewicht 1350 g/Tier | durchschnittl. Schlachtgewicht 1502 g/Tier | 152 g/Tier |
| 13100 Stall mit BHQ behandelt vornehmliches Krankheitsbild Diarrhoe | | | |

Die in einem Stall befindlichen Mastküken wurden zum einen mit Futtermittel (unbehandelt) und zum anderen mit Futtermittel und Zusatz (40 mg BHQ R11/kg Futter) vom 1. bis 28. Tag der Aufzucht täglich gefüttert. Das Ergebnis: Die unbehandelten Mastküken litten an einer wesentlich höheren Diarrhoerate als die mit BHQ R11 behandelten Masthähnchen und eindeutig an höheren Mast-(Schlacht-)Gewicht ermittelt werden konnte. Die Diarrhoe-Spezifikation ist trotz regulärer Futteraufnahme der Mastküken am Gewichtsverlust festzustellen.

## Tabelle 2: Wirksamkeit gegen Atemwegs- und/oder Lungenerkrankungen (Bronchialerkrankungen/Pneumonien); inhalative Gabe

| Stall Mastküken Anzahl | Schlachtergebnis g/Masthähnchen Behandlung 1. - 28. Tag | | Mehrgewicht durch BHQ-Behandlung |
|---|---|---|---|
| | unbehandelt | tägl. 10 ml BHQ R8 | |
| 28000 Stall unbehandelt | 1389 g/Tier | in 7 l Wasser mit Atomist pro 10.000 Mastküken (bei geschlossener Lüftung) in den Stall vernebelt | 94/g Tier |
| 25000 Stall mit BHQ behandelt | | 1483 g/Tier | |
| Vornehmliches Krankheitsbild Bronchitis, Pneumo-nie, letale Fälle | | | |

Die in so einem Stall befindlichen Mastküken wurden zum einen mit BHQ durch Vernebelung mit einem Atomist-Gerät in der Stallluft (1 x täglich, Vernebelung bei geschlossener Lüftung, 10 ml/10000Küken (1. bis 28. Tag) behandelt, der andere Fall blieb unbehandelt. Das Ergebnis: Die unbehandelten Mastküken litten unter hörbaren Atemgeräuschen, Lungen-LS-Entzündung, Pertonitis u. ä., was sich in geringerer Futteraufnahme darstellte, sodass ein Untergewicht durchschnittlich ermittelt werden konnte. Die BHQ-behandelten Mastküken waren gesünder und schwerer.

Beispiel 2

Wirksamkeit bei Krebserkrankungen (1 Probandentest)

orale Gabe BHQ bei sekundärem Karzinom (Knochentumor), osteoplastische Metastasen aus Mamma-Karzinom

Nach Mamma-OP entwickelten sich bei einer Patientin (43 Jahre) osteoplastische Metastasen, die als sekundäres Knochen(Tumor)-Karzinom konservativ mit Chemotherapie und Bestrahlung behandelt wurden (Level 1). Nach Absetzen der Zytostatika wurden wegen der Gefahr der Leukopenie die Bestrahlungen weiter gegeben unter oraler Gabe (kombinatorisch) von BHQ R15. Nach 3 Monaten trat eine subjektive Besserung wie auch der klinischen Daten ein (Level 2).

Tabelle 3

| Symptome | Level 0 | Level 1 | Level 2 |
|---|---|---|---|
| Generelle Gesundheit | - | - | +/- |
| Appetit | - | - | +/- |
| Schmerzen | - | - | - |

(-) negativ

Level 0 - Erkrankung

Level 1 - Behandlung: Chemotherapie/Bestrahlung

Level 2 - Behandlung: Bestrahlung unter oraler Gabe 3 - 5 g/Tag (3 Monate) BHQ R15

Tabelle 3   (fortgesetzt)

| Symptome | Level 0 | Level 1 | Level 2 |
|---|---|---|---|
| Fieber | + | + | - |
| Alkaline Phosphatase (ALP)* | 800 µg/100 ml | 280 µg/100 ml | 200 µg/100 ml |
| Leukozytose ** (Leukozytenvermehrung) | 22000 mm$^3$ | 14000 mm$^3$ | 10500 mm$^3$ |

\* ALP 40 190 normal

\*\* Leukozyten > 10000 mm$^3$ = Leukozytose (< 5.000 = Leukopenie (Leukozytenverminderung))

(-) negativ

(+) positiv

Level 0 - Erkrankung

Level 1 - Behandlung: Chemotherapie/Bestrahlung

Level 2 - Behandlung: Bestrahlung unter oraler Gabe 3 - 5 g/Tag (3 Monate) BHQ R15

Fazit: kombinatorisch begleitende BHQ-Gabe führte zur deutlichen Besserung und Wohlbefinden des Patienten.

Beispiel 3

Wirksamkeit bei Allergien (3 Probandentests)

Orale und Kontakthautpräparat (Externa)-BHQ-Gaben bei Neurodermitis Atopica (chron. rezidives Hautekzem "Eczema flexuarum" in den Gelenkbeugen bei Erwachesenen (32-40-46 Jahre).
Eine weitgehende Besserung im Vergleich zur "konservativen" Therapie konnte mit BHQ oral und äußerlich erzielt werden
3 Probanden litten unter rezidiven Hautekzemen in den Gelenkbeugen. Konservative Behandlung mit Antihistaminika und kortikoidhaltigen Salben brachten keine dauerhafte Linderung des Juckreizes und keine dauerhafte Eliminierung der ekzematösen Hautstellen (Level 0). Die konservative Therapie wurde orale Gabe von BHQ R13 30 Tage ersetzt. 2 Probanden besaßen keine sichtbaren Ekzeme mehr und spürten keinen Juckreiz mehr (Level 1). Die Probanden wurden dann noch mit BHQ-R7-Salbe äußerlich über 20 Tage an den Hautstellen 2 mal täglich behandelt. Alle Symptome waren obsolet (Level 2).

Tabelle 4

| Symptome Ekzem | Level 0 | Level 1 | Level 2 |
|---|---|---|---|
| 32 Jahre (w) | + | +/- | - |
| 40 Jahre (m) | + | - | - |
| 46 Jahre (m) | + | - | - |

+ Ekzeme vorhanden

+/- Besserung

- Ekzeme nicht mehr nachweisbar, Begleiterscheinungen obsolet

Level 0: Erkrankung, Behandlung symptomatisch mit Antihistaminika, Glukokortikoid und glukokortikoidhaltigem Externa
Level 1: Absetzen der konservativen Behandlung, 30 Tage orale Gabe mit BHQ R13 (1 x täglich 1,3 g)
Level 2: Behandlung nach 20 Tagen mit oraler Gabe von BHQ (Level 1) mit zusätzlichem Externa (Salbenpräparat) BHQ R7 Behandlung der ekzematösen Hautstellen über zwei Wochen (2 x täglich). 6 Monate danach traten keine Ekzeme mehr auf.

Beispiel 4

Antidepressive Wirksamkeit (2 Probandentests/weiblich 1/männlich 1)

Oft sind Erkrankungen wie Depressionen ausschließlich symptomatisch mit Psychopharmaka zu behandeln. Ursächlich können jedoch Mykosen/Mykotoxine die Auslöser derartiger Erkrankungen sein. Ziel war es, mit BHQ (s. auch Patentanmeldung EP 00124497) die gastrointestinale Überwucherung mit Aspergillus und Candida-(Hefe-)Pilzen zu Eliminieren und im Multi-Step-Verfahren BHQ R6 Regenerativum zu applizieren. Alle 3 Probanden waren nach Anwendung der BHQ-Therapie im Multi-Step depressionsfrei.

Tabelle 5

| Symptome | Level 0 | Level 1 | Level 2 |
|---|---|---|---|
| Generelle Gesundheit | - | +/- | + |
| Gewichtszunahme | + | +/- | - |
| Angstgefühle/depressiv part. Status | +/- | +/- | - |
| Mikrobiolog. gastrointestinaler Nachweis: | | | |
| Candida spec. | + | - | - |
| Aspergillus spec. | + | - | - |

- negativ

+ positiv

Level 0: Depression konservativ behandelt mit Antidepressiva (Seroxat®) Der generelle Gesundheitszustand wurde subjektiv als nicht zufriedenstellend berichtet, zeitweise traten zu verschiedenen Tageszeiten (z. B. abends) Depressionsschübe ein, sodass die Dosierungen erhöht wurden. Mikrobiologischer Nachweis auf Candida spec. und Aspergillus spec. war positiv. Die Probanden beklagten eine hohe Gewichtszunahme ("schwammiges Gefühl").

Level 1: 4 Wochen wurde oral täglich 1 x BHQ "aromahaltiges Arzneimittel" zur Dekontamination der Mykose und/oder ggf. der Mykotoxine gegeben (1 g/Tag). Laut Tabelle war eine Besserung bereits nach 4 Wochen festzustellen.

Level 2: Als nächster Schritt wurde nach Level 1 4 Wochen oral täglich (2 g) BHQ R6 Regenerativum gegeben. Die Probanden waren nach der Behandlung beschwerdefrei und reduzierten ihr Gewicht.

Beispiel 5

Wirksamkeit auf Krankheitsbilder, die durch Gallenresektion und Peritonitis entstehen mit defizitärer Produktion von Gallensäure und Enzymen. Ein Proband (weiblich) litt ständig unter Appetitlosigkeit, Unverträglichkeit nicht diätetischer Speisen, subjektiven, unspezifische Magen-Darm-Beschwerden und körperlicher und seelischer Belastbarkeit.

Tabelle 6

| Symptome | Level 0 | Level 1 |
|---|---|---|
| Generelle Gesundheit | - | + |
| Appetit | - | + |
| Speisenverträglichkeit | - | +/- |
| unspez. Magen-Darm-Beschwerden | - | + |
| körperliche Belastbarkeit | - | + |
| seelische Belastbarkeit | - | + |

- negativ

+ positiv

Level 0: Die Probandin litt unter den in Tabelle 6 aufgeführten Symptomen nach einer 1 Jahr zuvor absolvierten Gallenresektion und Peritonitis. Verschiedene konservative medikamentöse Gaben führten nicht zu einem subjektiv empfundenen, generellen, positiven Gesundheitszustand.

Level 1: BHQ R16 enthält als Regenerativum auch Enzyme. Die Therapie erfolgte oral mit täglichen Gaben (1,5 g) über einem Zeitraum von 90 Tagen. Die Probandin war nach diesem Zeitraum beschwerdenfrei.

Beispiel 6: Nahrungsmittel

Wirksamkeit: siehe Tabelle 7, Suspensionsversuche "BN 3" mit Vergleichszusammensetzung

Unter gastrointestinalen Bedingungen (37 °C) wächst nach einer Einwirkzeit von 6 Stunden die Keimzahl von 24.000 KBE/ml (kolonienbildende Einheiten/ml) mit Lactobacillus acidophilus auf 26.000 KBE/ml mit "BN 3" bei einer Dosierung von 0,1 %. Diese schwache Kolonisierung wird jedoch nach 24 Stunden Einwirkzeit zunichte gemacht, indem die Wachstumskontrolle (ohne "BN 3") auf 43.000 KBE/ml ansteigt, der mit "BN 3" behandelten Lactobacillus acidophilus jedoch um 90 % (log RF 1,0) auf 4.300 absinken, d. h. "BN 3" besitzt nach kurzzeitiger Stimulierung/ Kolonisierung nur kurz regenerative Eigenschaften, um dann seine Wirkung bei längerer Einwirkzeit (Verdauungsprozess ~ 16 - 24 h) zunichte zu machen und statt regenerativ dann sogar sehr stark dekontaminierend auf die einzubringenden "probiotischen Mikroorganismen" wirkt, also seine regenerativen Eingenschaften über einen Tag nicht erreicht. Die Idee dieser Präparate ist, durch Zugabe von Vitaminen und/oder Mineralstoffen das "Andocken" an die Mucosa-Rezepturen des gastrointestinalen Traktes von "probiotischen Mikroorganismen (z. B. der Gruppe Lactobacillus) durch Stimulierung oder Kolonisierung der Mikroorganismen zu erreichen. Dies gelingt hier nicht nach Beurteilung der gewählten Prüfkriterien mit oraler Eingabe einer einmaligen täglichen Dosis.

Tabelle 7: Quantitativer Suspensionsversuch

Testkeim: Lactobacillus acidophilus DSM 20079
KBE/ml: 3,7 • $10^7$
Testbedingungen: Testtemperatur 37 °C
Medium: MRS-Agar, MRS-Bouillon, pH 8,0 + Entn. IV (3 % Tween® + 0,3 % Lecithin + 0,1 % Histidin + 3 % Saponin)

| Produkt | Konz. in Vol.% | EWZ 6 h | Kontrolle 37 °C | EWZ 24 h | Kontrolle 37 °C |
|---------|----------------|---------|-----------------|----------|-----------------|
| BHQ R15 | 0,1 | 84.000 | 24.000 | 55.000 | 43.000 |
| BHQ R6 | 0,1 | 70.000 | 24.000 | 43.000 | 43.000 |
| BHQ R13 | 0,1 | 61.000 | 24.000 | 40.000 | 43.000 |
| BHQ R8 | 0,1 | 56.000 | 24.000 | 38.000 | 43.000 |
| BHQ R11 | 0,1 | 52.000 | 24.000 | 31.000 | 43.000 |
| BHQ R16 | 0,1 | 48.000 | 24.000 | 34.000 | 43.000 |
| BHQ R7 | 0,1 | 37.000 | 24.000 | 24.000 | 43.000 |
| BN3* | 0,1 | 26.000 | 24.000 | 4.300 | 43.000 |

\* Vergleichsbeispiel

Eine Zusammensetzung aus apathogenen Mikroorganismen und Vitaminen/Mineralien nach Stand der Technik "BN3" besitzt eine leichte Kolonisierungseffektivität nach 6 h (37 °C) Einwirkzeit, die innerhalb 24 h dekontaminative Wirkung, d. h. die umgekehrte Wirkung zeigt. Unter synergistischer Wirkung von Aroma, Enzym und Pflanzenextrakt und Vitaminen/Mineralstoffen kolonisiert der symbiontische Komplex aus apathogenen, fakultativ pathogenen und pathogenen Mikroorganismen über 6 und 24 Stunden (37 °C). Sobald Aromasynergismen mit Mikroorganismen symbiontisch wirken, zeigen sie selbst nach 24 h eine Kolonisierung, die über dem 6-h- bzw. 24-h-Kontrollwert liegt. Enzyme zeigen eine ähnliche, jedoch schwächere Wirkung.

**Patentansprüche**

**1.** Regeneratives Arzneimittel umfassend

(a) eine Mikroorganismuskomponente, enthaltend:

(a1) wenigstens einen Typ apathogener Mikroorganismen und/oder
(a2) wenigstens einen Typ pathogener oder fakultativ pathogener Mikroorganismen;

(b) eine Modulatorkomponente, enthaltend

(b1) wenigstens ein Enzym;
(b2) wenigstens einen GRAS(Generally Recognized As Safe)-Aromastoff oder ein Derivat davon und/oder

(b3) wenigstens einem tierischen oder pflanzlichen Extrakt oder ein Derivat davon, der/das eine Verbindung mit alkoholischer, Säure-, Ester, Aldehyd-, Acetal- , phenolischer, funktionellen Gruppen und/oder mit Doppelbindungen umfasst.

**2.** Arzneimittel nach Anspruch 1, wobei

der apathogene Mikroorganismus (a1) ein apathogener Lactobacillus ist und vorzugsweise ausgewählt ist aus Lactobacillus acidophilus, Lactobacillus bifidum, Lactobacillus bulgaris, Lactobacillus casei, Lactobacillus bifido longum und Lactobacillus fermentum und/oder ein Mikroorganismus, ausgewählt aus Pilzen, Dermatophyten, Hefe- und Sprosspilzen, die keine pathogenen oder toxinogenen Eigenschaften besitzen oder im Gastrointestinaltrakt vorkommen, einschließlich Sarcina, Staphylococcus epidermidis, Gaffkya, Streptococcus K-P, Corynebacterium xerosis, Corynebacterium hoffmanni, Bacillus subtilis, und Saccharomyces cervisiae; und/oder
die pathogenen oder fakultativ pathogenen Mikroorganismen (a2) ausgewählt sind aus Serratia marcescens, Alcaligenes faecalis, Citrobacter, Hafnia alvei, Afflya tetragena, Neisseria catarrhalis, Neisseria sicca, Neisseria Flavescens-, subflava-, flava-, perflva, Veillonella parvula, Borrelia buccalis, Rhodtorula rubra, Cladosporium, Geotrichum mucor, Mucor und Torulopsis,

wobei die Mikroorganismuskomponente vorzugsweise wenigstens zwei, besonders bevorzugt wenigstens drei verschiedene Mikroorganismen enthält.

**3.** Regeneratives Arzneimittel nach Anspruch 1 oder 2, wobei

das Enzym (b1) pflanzlichen, tierischen oder synthetischen Ursprungs ist und insbesondere ausgewählt ist aus Chymotrypsin, Bromelain, Papain, Pepsin, Trypsin, ein Enyzm aus Gruppen der Oxidoreduktasen, Dehydrogenasen, Oxygenasen, Transferasen, Hydrolasen, Esterasen, Glykosidasen, Proteasen, Lyasen, Isomerasen, Ligasen, Synthetasen, DNA-Ligasen, Amylasen, Lipasen, Phosphatasen, Leukoproteasen, Kathepsine, Oxidasen wie Katalasen und Peroxidasen, Carboxylasen ist und besonders bevorzugt eine Hydrolase oder Proteasen wie Bromelain, Trypsin, Ananain, Pepsin, Chymosin, Ficain ist; und/oder
der (Generally Recognized As Safe)-Aromastoff oder das Derivat davon (b2) ausgewählt sind aus:

(i) GRAS-Aroma-Alkohole oder deren Derivate, insbesondere Benzylalkohol, Acetoin, Ethylalkohol, Propylalkohol, iso-Propylalkohol, Propylenglykol, Glycerin, n-Butylalkohol, iso-Butylalkohol, Hexylalkohol, L-Menthol, Octylalkohol, Zimtalkohol, $\alpha$-Methylbenzylalkohol, Heptylalkohol, n-Amylalkohol, iso-Amylalkohol, Anisalkohol, Citronellol, n-Decylalkohol, Geraniol, $\beta$-$\gamma$-Hexanol, Laurylalkohol, Linalool, Nerolidol, Nonadienol, Nonylalkohol, Rhodinol, Terpineol, Borneol, Clineol, Anisol, Cuminylalkohol, 10-Undecen-1-ol, 1-Hexadecanol und deren Derivate;
(ii) Polyphenolverbindungen, insbesondere Brenzcatechin, Resorcin, Hydrochinon, Phloroglucin, Pyrogallol, Cyclohexan, Usninsäure, Acylpolyphenolen, Ligninen, Anthocyane, Flavonen, Catechinen, Gallussäurederivaten, Kaffesäure, Flavonoiden, Derivate der genannten Polyphenole und Extrakte aus Camellia Primula;
(iii) GRAS-Säuren oder Derivate, insbesondere Essigsäure, Aconitsäure, Adipinsäure, Ameisensäure, Apfelsäure, Capronsäure, Hydrozimtsäure, Pelagonsäure, Milchsäure, Phenoxyessigsäure, Phenylessigsäure, Valeriansäure, iso-Valeriansäure, Zimtsäure, Citronensäure, Mandelsäure, Weinsäure, Fumarsäure, Tanninsäure und deren Derivate;
(iv) GRAS-Phenole oder deren Derivate, insbesondere Thymol, Methyleugenol, Acetyleugenol, Safrol, Eugenol, Isoeugenol, Anethol, Phenol, Methylchavicol, Carvacrol, $\alpha$-Bisabolol, Fornesol, Anisol, Propenylguaethol und deren Derivate;
(v) GRAS-Ester, insbesondere Acetate einschließlich Iso-Amylacetat, Benzylacetat, Benzylphenylacetat, n-Butylacetat, Cinnamylacetat, Citronellylacetat, Ethylacetat, Eugenolacetat, Geranylacetat, Hexylacetat, Hydrocinnamylacetat, Linalylacetat, Octylacetat, Phenylethylacetat, Terpinylacetat, Triacetin, Kaliumacetat, Natriumacetat und Calciumacetat sowie Ester und Esterderivate der Säuren (iii);
(vi) Terpene, insbesondere Campher, Limonen und $\beta$-Caryophyllen;
(vii) Acetale, insbesondere Acetal, Acetaldehyddibutylacetal, Acetaldehyddipropyl-acetal, Acetaldehydphenethylpropylacetal, Zimtaldehydethylenglycolacetal, Decanaldimethylacetal, Heptanaldimethylacetal, Heptanalglycerylacetal und Benzaldehydpropylenglykolacetal;
(viii) Aldehyde, insbesondere Acetylaldehyd, Anisaldehyd, Benzaldehyd, iso-Butylaldehyd, Citral, Citronellal, n-Caprinaldehyd, Ethylvanillin, Fufural, Heliotropin, Heptylaldehyd, Hexylaldehyd, 2-Hexenal, Hydrozimtaldehyd, Laurylaldehyd, Nonylaldehyd, Octylaldehyd, Phenylacetaldehyd, Propionaldehyd, Vanillin, Zimtaldehyd, Perillaaldehyd und Cuminaldehyd;

(ix) GRAS-etherischen Öle, insbesondere etherische Öle und/oder alkoholische, glykolische oder durch $CO_2$-Hochdruckverfahren erhaltene Extrakte der folgenden Pflanzen

(ix1) Öle bzw. Extrakte mit hohem Anteil an Alkoholen: Melisse, Koriander, Kardamon, Eukalyptus;

(ix2) Öle bzw. Extrakte mit hohem Anteil an Aldehyden: Eukalyptus citriodora, Zimt, Zitrone, Lemongras, Melisse, Citronella, Limette, Orange;

(ix3) Öle bzw. Extrakte mit hohem Anteil an Phenolen: Oreganum, Thymian, Rosmarin, Orange, Nelke, Fenchel, Campher, Mandarine, Anis, Cascarille, Estragon und Piment;

(ix4) Öle bzw. Extrakte mit hohem Anteil an Acetaten: Lavendel;

(i5) Öle bzw. Extr akte mit hohem Anteil an Estern: Senf, Zwiebel, Knoblauch;

(ix6) Öle bzw. Extrakte mit hohem Anteil an Terpenen: Pfeffer, Pomeranze, Kümmel, Dill, Zitrone, Pfefferminz, Muskatnuss; und/oder

der tierische oder pflanzliche Extrakt (b3) ausgewählt ist aus Extrakten von Aloe, Annatto (Samen), Arnika, Baldrian, Beinwell, Birkenblätter, Blütenpollen, Brennessel, Champignon, Dill, Efeu, Eibisch, Eichen(rinde), Fichten(nadeln), Gingko(blätter), Ginseng(wurzel), Hamamelis(blätter) Henna, Heublumen, Hibiskus, Hopfen, Huflattich, Ingwer(wurzel), Johanniskraut, Kakao, Kaffee, Kamille, Karotten, Knoblauch, Kolanuss, Koriander, Kümmel, Lavendel, Linden(blüten), Löwenzahn, Malven, Mäusedorn, Majoran, Melisse, Orangen(schalen), Orthosiphonis, Paprika, Pfeffer, Pfefferminze, Pilze, Rhabarber, Ringelblumen, Rosmarin, Rosskastanien, Salbei, Schachtelhalm, Schöllkraut, Schwarztee, Süssholz, Wacholder(beeren), Weißdorn, Weizenkleie, Zimt, Algen, Banane, Benzo, Birkenteer, Bochshorn, Boldo, Chinarinde, Copai-Balsam, Derriswurzel, Kajeputöl, Enzian, Eukalyptus, Fenchel, Fieberklee, Kalmus, Kartoffelstärke, Kiefer, Kolophonium, Zwiebel, Lärchenterpentin, Leinsamen, Lorbeer, Nadelholzteer, Niduli-Baum, Perubalsam, Pyrethrum, Quassiaholz, Reis, Rotenon, Sadebaum, Saponin, Schwarzwurzel, Senf, Sesam, Soja, Sonnenhut, Tausendgüldenkraut, Terpentinöl, Wegerich, weiße Nieswurz, Wermut, Zypresse, Apfel, Avocado, Theobroma grandiflorum, Grapefruit, Guarana, Gurke, Hafer, Melone, Jojoba, Kiwi, Klette, Klee, Kokosnuss, Kornblume, Mandel, Mango, Mistel, Taxus, Papaya, Passionsfrucht, Pfirsich, Schafgarbe, Schlüsselblume, Sonnenblume, Taubnessel, Thymian, Walnuss, Wasserminze, Ylang Ylang, Cochenille, Bienenwachs, Gelee Royal, Propolis, Schelllack, Lecithin, Formiate, Ameisensäure, Lutein, Canthaxanthin, Riboflavin, Lactoflavin, Speisefettsäuren und -derivate, Angora, Astrahan, Cashmere, Mohair, Wolle, Milch, Kasein, Kaseinogen, Bienenpollen, Bienengift, Honig, Molke, Eier, Karatin, Plazenta, Fisch, Fleisch, Geflügel, Ambra, Capiz, Kaviar, Chitin, Koralle, Squalene, Seehundöl, Walöl, Schwämme, Perlen, Rogen, Robbe, Schalentiere, Spermöl, Samenöl, Wachs, Karmine, Karminsäure, Catgut, Moschus, Zibet, Rizinus, Östrogen, Progesteron, Testeron, Hormone, Elfenbein, Lanolin, Nerzöl, Pergament, Seide, Schlangengift, Harnstoff, Aminosäuren, Aspik, Knochen, Borsten, Kollagen, Daunen, Blut, Gelatine, Glycerin, Fell, Leder, Oleostearin, Lab, Stearinderivate.

4.  Arzneimittel nach einem oder mehreren der Ansprüche 1 bis 3, wobei das Arzneimittel weiterhin eine Zusatzstoffkomponente (c) enthält, die ausgewählt ist aus

(c1) wenigstens einem Vitamin,
(c2) wenigstens einem Mineralstoff oder Spurenelement,
(c3) wenigstens einem Wachstumsförderer oder Supporter,
(c4) wenigstens einem Zusatzstoff insbesondere Trägerstoff, Trennmittel oder Timersubstanz,
(c5) wenigstens einem Enzymzusatzstoff,

wobei vorzugsweise

die Vitamine (c1) ausgewählt sind aus Ascorbinsäure, Ascorbinpalmitat, Ascorbate, insbesondere Magnesiumascorbate, β-, γ- und δ-Tocopherol, α-und β-Carotin, Thiamin, Riboflavin, Niacin, Pantothensäure, Calciumpantothenat, Pyridoxinhydrochlorid, Cyanocobalamin, Col-Calciferol-Cholerterin, Biotin, Folsäure und Nicotinamid; und/oder
die Mineralstoffe und Spurenelemente (c2) ausgewählt sind aus Calcium, Phosphor, Eisen, Magnesium, Iod, Kalium, Chlor, Kupfer, Mangan, Chrom, Molybdän, Selen, Silicium und Zinn; und/oder
die Wachstumsförderer und Supporter (c3) ausgewählt sind aus Hefe, Zucker, insbesondere Milch-, Trauben-, Rüben- und Rohrzucker, Gelee Royale, Pepton, Gallensalze, Kochsalz, Agar, Gelatine, Kartoffelextrakt, Fleischextrakt, Citrate, Rinderherzgewebe, Stärke, Nikotinsäure, Tryptose, Natriumazid, Mannit, Magnesiumsulfat, Dinatriumhydrogenphosphat, Kaliumhydrognphosphat, NatriumIpyruvat, Caseinpepton, Glycin, Lithiumchlorid, Dikaliumphosphat, Natriumsulfit, Eisensulfat, Proteose-Pepton, Leber-Verdauungprodukt, Kartoffelinfus, Ochsengalle, Natriumthioglycolat, Calciumchlorid, Sojamehl, Sojamehl-Pepton, L-Cystein, L-Asparagin, Natriumdithionit, Lecithin, Mineralstoffe und deren Salze, Rinder- und Schafsserum, Desoxyribonukleinsäuren, Acetate, Adeninsulfat, Guaninhydrochlorid, Caseinhydrolysat, Kalbshirninfusion, Rinderherzinfusion, Holzkohle und Leberinfusion; und/oder

die Zusatzstoffe (c4) ausgewählt sind aus Salzen, Zucker, Kohlenhydraten, Eiweiß, Proteinen, Flüssigkeiten, insbesondere Ölen und Wasser, Fette, Fettsäuren, und Trägerstoffen, insbesondere Propylenglycol, Benzylalkohol, Glycerin, Alginate, Lactate, Kieselsäure, Gelatine und Agar; und/oder

die Enzymzusatzstoffe (c5) ausgewählt sind aus Inisitol, Rutin, Lutein, L-Arginin, L-Cystein, L-Karnitin, L-Lysin, L-Prolin und Coenzym Q10.

5. Arzneimittel nach einem oder mehreren der Ansprüche 1 bis 4, wobei die Mikroorganismuskomponente (a) wenigstens einen apathogenen Mikroorganismus (a1), vorzugsweise Lactobacillus acidophilus und/oder Lactobacillus fermentum, enthält, die Modulatorkomponente (b) wenigstens ein Enzym (b1), vorzugsweise eine Hydrolase und/oder Protease, enthält, und das Arzneimittel gegebenenfalls noch eine Zusatzstoffkomponente (c), ausgewählt aus Vitaminen (c1), Mineralstoffen und Spurenelementen (c2) enthält.

6. Arzneimittel nach einem oder mehreren der Ansprüche 1 bis 4, wobei die Mikroorganismuskomponente (a) wenigstens einen apathogenen Mikroorganismus (a1), vorzugsweise Lactobacillus acidophilus und/oder Lactobacillus fermentum, enthält, die Modulatorkomponente (b) wenigstens einen GRAS-Aromastoff oder ein Derivat davon (b2), vorzugsweise Kardamon, Anis und/oder Tannin, enthält, und das Arzneimittel gegebenenfalls noch eine Zusatzstoffkomponente (c), ausgewählt aus Vitaminen (c1), Mineralstoffen und Spurenelementen (c2), und/oder ein oder mehrere Enzyme (b1) enthält.

7. Arzneimittel nach einem oder mehreren der Ansprüche 1 bis 4, wobei die Mikroorganismuskomponente (a) wenigstens einen apathogenen Mikroorganismus (a1), vorzugsweise Lactobacillus acidophilus und/oder Lactobacillus fermentum, enthält, die Modulatorkomponente (b) wenigstens einem tierischen oder pflanzlichen Extrakt oder ein Derivat davon (b3), vorzugsweise ein Extrakt von Taxus, Gingko, Avocado und/oder Bienenpollen, enthält, und das Arzneimittel gegebenenfalls noch eine Zusatzstoffkomponente (c), ausgewählt aus Vitaminen (c1), Mineralstoffen und Spurenelementen (c2), ein oder mehrere Enzyme (b1) und/oder einen oder mehrere GRAS-Aromastoffe oder Derivate davon (b2) enthält.

8. Arzneimittel nach einem oder mehreren der Ansprüche 1 bis 4, wobei die Mikroorganismuskomponente (a) wenigstens einen pathogenen oder fakultativ pathogenen Mikroorganismus (a2), vorzugsweise Bacillus subtilis, Serratia marcescens und/oder Streptococcus, enthält, eine oder mehrere Modulatorkomponenten (b) und optionale Zusatzstoffkomponenten (c), wie in Ansprüchen 5 bis 7 definiert, enthält und optional einen apathogenen Mikroorganismus (a1) enthält.

9. Arzneimittel nach einem oder mehreren der Ansprüche 1 bis 8, wobei die Zahl der Mikroorganismen (a) im Arzneimittel von $10^1$ bis $10^{20}$, vorzugsweise $10^6$ bis $10^{14}$ pro g oder pro ml Arzneimittel beträgt und/oder der Gehalt an (b) und (c) insgesamt 1 µg bis 200 mg, vorzugsweise jeweils 10 µg bis 80 mg pro g oder pro ml Arzneimittel beträgt.

10. Arzneimittel nach einem oder mehreren der Ansprüche 1 bis 9, das weiterhin ein oder mehrwertige Alkohole mit 2 bis 10 C-Atomen, Emulgatoren, Stabilisatoren, Antioxidantien, Konservierungsmittel, Lösemittel und/oder Trägerstoffe enthält.

11. Arzneimittel nach einem oder mehreren der Ansprüche 1 bis 10, wobei das Arzneimittel inhalativ, oral, intravenös, intramuskulär, rektal, Kontaktpräparat, innerlich/äußerlich, intraperitoneal, subkutan, auf/in inneren Organen in Form von Tabletten, flüssig, Gas, Pulver, Injektion, Infusion, Suppositorium, Spray, Salben oder Pflaster appliziert werden kann, und/oder wobei das Arzneimittel ein Immunregulator, Immunregenerativum, Antiinfektivum, Antimykotikum, Antitoxinozidikum Antiresistenzmittel und/oder Zytostatikum ist.

12. Verwendung einer Zusammensetzung aus Mikroorganismuskomponente (a) und Modulatorkomponente (b) wie in Ansprüchen 1 bis 8 definiert , zur Herstellung eines dekontaminativen und/oder regenerativen Mittels, insbesondere eines Immunregulators, Immunregenerativums, Antiinfektivums, Antimykotikums, Antitoxinozidikums Antiresistenzmittels oder Zytostatikums, zur Behandlung von Mensch und Tier.

13. Verfahren zur Behandlung von unspezifischer Genese, Allergien, Krebserkrankungen, Symptomen, die ursächlich nicht regenerativ therapierbar waren, von Immunerkrankungen, infektiösen Erkrankungen und Erkrankungen im Gastrointestinaltrakt bei Mensch und Tier, umfassend Verabreichen einer Zusammensetzung aus Mikroorganismuskomponente (a) und Modulatorkomponente (b), wie in einem oder mehreren der Ansprüchen 1 bis 8 definiert.

14. Nahrungsmittel, Nahrungsergänzungsmittel, Tiernahrungsmittel oder Tiernahrungsergänzungsmittel, umfassend eine Zusammensetzung aus Mikroorganismuskomponente (a) und Modulatorkomponente (b), wie in einem oder mehreren der Ansprüche 1 bis 8 definiert.

15. Nahrungsmittel, Nahrungsergänzungsmittel, Tiernahrungsmittel oder Tiernahrungsergänzungsmittel nach einem oder mehreren der Ansprüche 14, enthaltend 0,1 bis 900 mg, vorzugsweise 1 bis 200 mg der Zusammensetzung aus (a), (b) und (c) pro g Nahrungsmittel, Nahrungsergänzungsmittel, Tiernahrungsmittel oder Tiernahrungsergänzungsmittel.

# EP 1 228 769 A1

<table>
<tr><td colspan="2"><strong>Europäisches Patentamt</strong></td><td colspan="2"><strong>EUROPÄISCHER TEILRECHERCHENBERICHT</strong><br>der nach Regel 45 des Europäischen Patent–<br>übereinkommens für das weitere Verfahren als<br>europäischer Recherchenbericht gilt</td><td><strong>Nummer der Anmeldung</strong><br>EP 01 10 2384</td></tr>
</table>

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.7) |
|---|---|---|---|
| X,D | DE 198 30 528 A (MERCK PATENT GMBH)<br>1. Juli 1999 (1999-07-01)<br>* Ansprüche * | 1 | A61K45/06<br>A61P43/00 |
| A | DE 39 38 233 A (HEPPNER NORBERT)<br>25. Oktober 1990 (1990-10-25)<br>* Ansprüche * | 1-15 | |
| A | US 6 080 401 A (PRASAD NARAPARAJU A V ET AL) 27. Juni 2000 (2000-06-27)<br>* Zusammenfassung * | 1-15 | |
| A | WO 97 29762 A (PROCTER & GAMBLE)<br>21. August 1997 (1997-08-21)<br>* Ansprüche * | 1-15 | |
| A | US 5 741 494 A (ARAKI SEIICHI ET AL)<br>21. April 1998 (1998-04-21)<br>* Ansprüche * | 1-15 | |

**RECHERCHIERTE SACHGEBIETE (Int.Cl.7)**

A61K

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| DEN HAAG | 15. August 2001 | Leherte, C |

| KATEGORIE DER GENANNTEN DOKUMENTEN | |
|---|---|
| X : von besonderer Bedeutung allein betrachtet<br>Y : von besonderer Bedeutung in Verbindung mit einer anderen Veröffentlichung derselben Kategorie<br>A : technologischer Hintergrund<br>O : nichtschriftliche Offenbarung<br>P : Zwischenliteratur | T : der Erfindung zugrunde liegende Theorien oder Grundsätze<br>E : älteres Patentdokument, das jedoch erst am oder nach dem Anmeldedatum veröffentlicht worden ist<br>D : in der Anmeldung angeführtes Dokument<br>L : aus anderen Gründen angeführtes Dokument<br>.......................<br>& : Mitglied der gleichen Patentfamilie, übereinstimmendes Dokument |

EPO FORM 1503 03.82 (P04C09)

Europäisches
Patentamt

**UNVOLLSTÄNDIGE RECHERCHE
ERGÄNZUNGSBLATT C**

Nummer der Anmeldung

EP 01 10 2384

Obwohl die Ansprüche 13 sich auf ein Verfahren zur Behandlung des menschlichen/tierischen Körpers bezieht (Artikel 52(4) EPÜ), wurde die Recherche durchgeführt und gründete sich auf die angeführten Wirkungen der Verbindung/Zusammensetzung.

---

Unvollständig recherchierte Ansprüche:
1-15

Grund für die Beschränkung der Recherche:

Die geltenden Patentansprüche 1-15 beziehen sich auf eine unverhältnismäßig große Zahl möglicher Zusammensetzungen, von denen sich nur ein kleiner Anteil im Sinne von Art. 84 EPÜ auf die Beschreibung stützen und/oder als im Sinne von Art. 83 EPÜ in der Patentanmeldung offenbart gelten kann. Im vorliegenden Fall fehlt den Patentansprüchen die entsprechende Stütze und fehlt der Patentanmeldung die nötige Offenbarung in einem solchen Maße, daß eine sinnvolle Recherche über den gesamten erstrebten Schutzbereich unmöglich erscheint. Daher wurde die Recherche auf die Teile der Patentansprüche gerichtet, welche im o.a. Sinne als gestützt und offenbart erscheinen, nämlich die Teile betreffend, die Zusammensetzungen wie sie in den Ausführungsbeispielen auf Seiten 29-31 angegeben sind.

## ANHANG ZUM EUROPÄISCHEN RECHERCHENBERICHT
## ÜBER DIE EUROPÄISCHE PATENTANMELDUNG NR.

EP 01 10 2384

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-08-2001

| Im Recherchenbericht angeführtes Patentdokument | Datum der Veröffentlichung | Mitglied(er) der Patentfamilie | Datum der Veröffentlichung |
|---|---|---|---|
| DE 19830528 A | 01-07-1999 | EP 0931543 A<br>JP 11199496 A<br>US 6254886 B | 28-07-1999<br>27-07-1999<br>03-07-2001 |
| DE 3938233 A | 25-10-1990 | KEINE | |
| US 6080401 A | 27-06-2000 | AU 1737000 A<br>WO 0029007 A | 05-06-2000<br>25-05-2000 |
| WO 9729762 A | 21-08-1997 | AU 1758197 A<br>CA 2245810 A<br>CN 1211188 A<br>EP 0880354 A<br>JP 11504048 T | 02-09-1997<br>21-08-1997<br>17-03-1999<br>02-12-1998<br>06-04-1999 |
| US 5741494 A | 21-04-1998 | CA 2159359 A<br>EP 0691848 A<br>WO 9422459 A<br>JP 8509211 T | 13-10-1994<br>17-01-1996<br>13-10-1994<br>01-10-1996 |

EPO FORM P0461

Für nähere Einzelheiten zu diesem Anhang : siehe Amtsblatt des Europäischen Patentamts, Nr.12/82